# EUROPEAN PATENT APPLICATION

(11) **EP 1 559 706 A1**
(43) Date of publication of application: **03.08.2005**
(21) Application number: 03754057.2
(22) Date of filing: 09.10.2003
(51) Int. Cl.: C07C 211/58, C09K 11/06, H05B 33/14, H05B 33/22

(54) **AROMATIC AMINE DERIVATIVE AND ORGANIC ELECTROLUMINESCENT ELEMENT EMPLOYING THE SAME**

(30) Priority: 06.11.2002 JP 2002322545
(71) Applicant: IDEMITSU KOSAN CO., LTD., Tokyo 100-8321 (JP)
(72) Inventor: KAWAMURA, Hisayuki, Sodegaura-shi, Chiba 299-0205 (JP); HOSOKAWA, Chishio, Sodegaura-shi, Chiba 299-0205 (JP)
(74) Representative: Gille, Struck, Neidlein, Prop, Roos
(86) International application number: PCT/JP2003/012977
(87) International publication number: WO 2004/041774

(57) **Abstract**

Provided are an aromatic amine compound represented by the following Formula (1) and an organic electroluminescent element which has at least one organic thin film layer containing the above aromatic amine derivative in the form of a single component. The organic electroluminescent element described above has a high luminescent efficiency even at a low voltage and a long life. It can emit blue light even at high temperatures. In Formula (1), Ar¹ and Ar² each represent naphthyl and the like; Ar³ to Ar⁶ each represent phenyl, naphthyl, phenanthryl and the like; Ar⁷ to Ar¹⁰ each represent 1,4-phenylene and the like; L represents a single bond and the like; provided that the conditions of (1) and/or (2) are satisfied:
(1) at least one of Ar³ to Ar⁶ is a condensed aryl group having 10 to 50 nuclear carbon atoms and
(2) at least one of Ar¹ and Ar² is a condensed aryl group having 12 to 50 nuclear carbon atoms.

## Description

### THECNICAL FIELD

The present invention relates to an aromatic amine derivative and an organic electroluminescent element making use of the same, more specifically to an organic electroluminescent element which can emit blue light under a high temperature while maintaining a high luminous efficiency even at a low voltage and a long life and an aromatic amine derivative which materializes the same.

### RELATED ART

An organic electroluminescent (EL) element is a spontaneous light emitting element making use of the principle that a fluorescent substance emits light by recombination energy of holes injected from an anode and electrons injected from a cathode when an electric field is applied. Since a low voltage-driven organic EL element of a laminate type was reported by C. W. Tang of Eastman Kodak Company (C. W'. Tang and S. A. Vanslyke, Applied Physics Letters, Vol. 51, p. 913, 1987), researches on organic EL elemens comprising organic materials as structural materials have actively been carried out. Tang et al. use tris(8-hydroxyquinolinol)aluminum for the light emitting layer and a triphenyldiamine derivative for the hole transporting layer. The advantages of a laminate structure include that an efficiency of holes injected into a light emitting layer can be elevated, that a forming efficiency of excited particles formed by blocking electrons injected from a cathode to recombine them can be raised and that excited particles formed in the light emitting layer can be shut up. As shown in the above example, a two layer type comprising a hole transporting (injecting) layer and an electron transporting and light emitting layer and a three layer type comprising a hole transporting (injecting) layer, a light emitting layer and an electron transporting (injecting) layer are well known as the element structure of the organic EL element. In such laminate type structural element, an element structure and a forming method are studied in order to enhance a recombination efficiency of holes and electrons injected.

A hole-injecting material used for the above organic EL element includes, for example, high molecular weight aromatic amine compounds disclosed in Japanese Patent Application Laid-Open No. 301934/1997, triarylamine polymers disclosed in International Patent Publication No. 30071/1998 and phenylenediamine derivatives disclosed in Japanese Patent Application Laid-Open No. 309566/2000. All of the above compounds have a small ionization potential and therefore holes are liable to be injected from an anode. In addition thereto, they have a higher hole mobility than those of star burst amine derivatives disclosed in Japanese Patent Application Laid-Open No. 308688/1992 and are suited as a hole-injecting material.

However, organic EL elements prepared by using the above hole injecting materials do not have a satisfactory heat resistance, and they can not maintain blue light emission particularly when exceeding 130°C and are unsuitable to on-vehicle uses to which a heat resistance is required.

### DISCLOSURE OF THE INVENTION

The present invention has been made in order to solve the problems described above, and an object thereof is to provide an organic electroluminescent element which can emit blue light under a high temperature while maintaining a high luminous efficiency even at a low voltage and a long life and an aromatic amine derivative which materializes the same.

Intensive researches repeated by the present inventors in order to achieve the object described above have resulted in finding that the object described above can be achieved by using a novel aromatic amine compound having a specific structure represented by the following Formula (1) as a material for an organic EL element and using it particularly as a hole injecting material, and thus they have come to complete the present invention.

That is, the present invention provides a novel aromatic amine derivative represented by the following Formula (1): 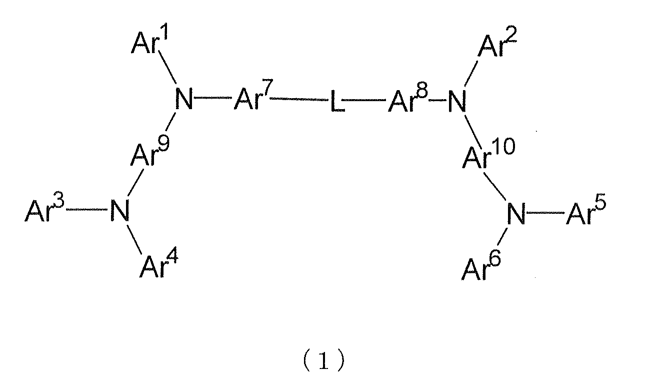 wherein Ar¹ to Ar² each represent a substituted or non-substituted condensed aryl group having 10 to 50 nuclear carbon atoms; Ar³ to Ar⁶ each represent a substituted or non-substituted aryl group having 6 to 50 nuclear carbon atoms; Ar⁷ to Ar¹⁰ each represent a substituted or non-substituted arylene group having 6 to 50 nuclear carbon atoms; substituents of Ar⁷ and Ar⁸ may form a ring;
L represents a single bond, an ether bond, a thioethers bond, a substituted or non-substituted arylene group having 6 to 50 nuclear carbon atoms, a substituted or non-substituted heteroarylene group having 5 to 50 nuclear carbon atoms, a substituted or non-substituted alkylene group having 1 to 50 carbon atoms or a substituted or non-substituted alkylidene group having 2 to 50 carbon atoms;
provided that the conditions of (1) and/or (2) are satisfied:
(1) at least one of Ar³ to Ar⁶ is a substituted or non-substituted condensed aryl group having 10 to 50 nuclear carbon atoms and
(2) at least one of Ar¹ to Ar² is a substituted or non-substituted condensed aryl group having 12 to 50 nuclear carbon atoms.

Further, the present invention provides an organic EL element in which an organic thin film layer comprising a single layer or plural layers including at least a light emitting layer is interposed between a cathode and an anode, wherein at least one layer of the above organic thin film layers contains the aromatic amine derivative described above in the form of a single component or a mixed component.

### BEST MODE FOR CARRYING OUT THE INVENTION

The aromatic amine derivative of the present invention comprises the compound represented by Formula (1) described above.

In Formula (1), Ar¹ to Ar² each represent a substituted or non-substituted condensed aryl group having 10 to 50 nuclear carbon atoms. To be specific, they include 1-naphthyl, 2-naphthyl, 1-anthryl, 2-anthryl, 9-anthryl, 1-phenanthryl, 2-phenanthryl, 3-phenanthryl, 4-phenanthryl, 9-phenanthryl, 1-naphthacenyl, 2-naphthacenyl, 9-naphthacenyl, 1-pyrenyl, 2-pyrenyl, 4-pyrenyl, 3-methyl-2-naphthyl, 4-methyl-1-naphthyl and 4-methyl-1-anthryl, and 1-naphthyl, 2-naphthyl, 9-phenanthryl, 1-naphthacenyl, 2-naphthacenyl, 9-naphthacenyl, fluoranthenyl are preferred.

Ar³ to Ar⁶ each represent a substituted or non-substituted aryl group having 6 to 50 nuclear carbon atoms. To be specific, they include phenyl, 1-naphthyl, 2-naphthyl, 1-anthryl, 9-anthryl, 1-phenanthryl, 2-phenanthryl, 3-phenanthryl, 4-phenanthryl, 9-phenanthryl, 1-naphthacenyl, 2-naphthacenyl, 9-naphthacenyl, 1-pyrenyl, 2-pyrenyl, 4-pyrenyl, 2-biphenylyl, 3-biphenylyl, 4-biphenylyl, p-terphenyl-4-yl, p-terphenyl-3-yl, p-terphenyl-2-yl, m-terphenyl-4-yl, m-terphenyl-3-yl, m-terphenyl-2-yl, o-tolyl, m-tolyl, p-tolyl, p-t-butylphenyl, p-(2-phenylpropyl)phenyl, 3-methyl-2-naphthyl, 4-methyl-1-naphthyl, 4-methyl-1-anthryl, 4'-methylbiphenylyl, 4"-t-butyl-p-terphenyl-4-yl and fluoranthenyl, and preferred are phenyl, 1-naphthyl, 2-naphthyl, 9-phenanthryl, 1-naphthacenyl, 2-naphthacenyl, 9-naphthacenyl, 1-pyrenyl, 2-pyrenyl, 4-pyrenyl, 2-biphenylyl, 3-biphenylyl, 4-biphenylyl, o-tolyl, m-tolyl, p-tolyl and p-t-butylphenyl.

Ar⁷ to Ar¹⁰ each represent a substituted or non-substituted arylene group having 6 to 50 nuclear carbon atoms. To be specific, they include 1,4-phenylene, 1,4-naphthylene, 1,4-anthracenylene, 9,10-anthracenylene, and 1,4-phenylene is preferred.

The substituents of Ar⁷ to Ar⁸ may form a ring, and it includes, for example, saturated five-membered rings. The substituents forming the ring include tetramethylene, pentamethylene, hexamethylene, diphenylmethane-2, 2'-diyl, diphenylethane-3, 3'-diyl and diphenylpropane-4, 4'-diyl.

L represents a single bond, an ether bond, a thioethers bond, a substituted or non-substituted arylene group having 6 to 50 nuclear carbon atoms, a substituted or non-substituted heteroarylene group having 6 to 50 nuclear carbon atoms, a substituted or non-substituted alkylene group having 1 to 50 carbon atoms or a substituted or non-substituted alkylidene group having 2 to 50 carbon atoms.

The arylene group having 6 to 50 nuclear carbon atoms includes, for example, 1,4-phenylene, 1,4-naphthylene, 1,4-anthracenylene and 9,10-anthracenylene, and 1,4-phenylene and 1,4-naphthylene are preferred.

The heteroarylene group having 6 to 50 nuclear carbon atoms includes, for example, pyrrolylene, furanylene, thiophenylene, silolylene, pyridylene, imidazolylene, pyrimidylene, carbazolylene, selenophenylene, oxadiazolylene and triazolylene, and thiophenylene and carbazolylene are preferred.

The substituted, non-substituted alkylene group having 1 to 50 carbon atoms includes, for example, methylene, ethylene, propylene, isopropylene, n-butylene, s-butylene, isobutylene, t-butylene, n-pentylene, n-hexylene, n-heptylene, n-octylene, hydroxymethylene, 1-hydroxyethylene, 2-hydroxyethylene, 2-hydroxyisobutylene, 1,2-dihydroxyethylene, 1,3-dihydroxyisopropylene, 1,2,3-trihydroxypropylene, chloromethylene, 1-chloroethylene, 2-chloroethylene, 2-chloroisobutylene, 1,2-dichloroethylene, 1,3-dichloroisopropylene, 1,2,3-trichloropropylene, bromomethylene, 1-bromoethylene, 2-bromoethylene, 2-bromoisobutylene, 1,2-dibromoethylene, 1,3-dibromoisopropylene, 1,2,3-tribromopropylene, iodomethylene, 1-iodoethylene, 2-iodoethylene, 2-iodoisobutylene, 1,2-diiodoethylene, 1,3-diiodoisopropylene, 1,2,3-triiodopropylene, aminomethylene, 1-aminoethylene, 2-aminoethylene, 2-aminoisobutylene, 1,2-diaminoethylene, 1,3-diaminoisopropylene, 1,2,3-triaminopropylen, cyanomethylene, 1-cyanoethylene, 2-cyanoethylene, 2-cyanoisobutylene, 1,2-dicyanoethylene, 1,3-dicyanoisopropylene, 1,2,3-tricyanopropylen, nitromethylene, 1-nitroethylene, 2-nitroethylen, 2-nitoroisobutylen, 1,2-dinitroethylene, 1,3-dinitroisopropylene, 1,2,3-trinitropropylen, cyclopropylene, cyclobutylene, cyclopentylene, cyclohexylene, 4-methylcyclohexylene, adamantylene and norbornylene, and methylene is preferred.

The alkylidene group having 2 to 50 carbon atoms includes, for example, propylidene, isopropylidene, butylidene, pentylidene, cyclopentylidene and cyclohexylidene, and cyclohexylidene is preferred.

The substituents of Ar¹ to Ar¹⁰ and L are a substituted or non-substituted aryl group having 6 to 50 nuclear carbon atoms, a substituted or non-substituted aromatic heterocycle group having 5 to 50 nuclear carbon atoms, a substituted or non-substituted alkyl group having 1 to 50 carbon atoms, a substituted or non-substituted alkoxy group having 1 to 50 carbon atoms, a substituted or non-substituted aralkyl group having 1 to 50 carbon atoms, a substituted or non-substituted aryloxy group having 5 to 50 nuclear carbon atoms, a substituted or non-substituted arylthio group having 5 to 50 nuclear carbon atoms, a substituted or non-substituted alkoxycarboxyl group having 1 to 50 carbon atoms, a halogen atom, a cyano group, a nitro group and a hydroxyl group.

The examples of the substituted or non-substituted aryl group having 6 to 50 nuclear carbon atoms include phenyl, 1-naphthyl, 2-naphthyl, 1-anthryl, 2-anthryl, 9-anthryl, 1-phenanthryl, 2-phenanthryl, 3-phenanthryl, 4-phenanthryl, 9-phenanthryl, 1-naphthacenyl, 2-naphthacenyl, 9-naphthacenyl, 1-pyrenyl, 2-pyrenyl, 4-pyrenyl, 2-biphenylyl, 3-biphenylyl, 4-biphenylyl, p-terphenyl-4-yl, p-terphenyl-3-yl, p-terphenyl-2-yl, m-terphenyl-4-yl, m-terphenyl-3-yl, m-terphenyl-2-yl, o-tolyl, m-tolyl, p-tolyl, p-t-butylphenyl, p-(2-phenylpropyl)phenyl, 3-methyl-2-naphthyl, 4-methyl-1-naphthyl, 4-methyl-1-anthryl, 4'-methylbiphenylyl, 4"-t-butyl-p-terphenyl-4-yl and fluoranthenyl.

The examples of the substituted or non-substituted aromatic heterocycle group having 5 to 50 nuclear carbon atoms include 1-pyrrolyl, 2-pyrrolyl, 3-pyrrolyl, pyrazinyl, 2-pyridinyl, 3-pyridinyl, 4-pyridinyl, 1-indolyl, 2-indolyl, 3-indolyl, 4-indolyl, 5-indolyl, 6-indolyl, 7-indolyl, 1-isoindolyl, 2-isoindolyl, 3-isoindolyl, 4-isoindolyl, 5-isoindolyl, 6-isoindolyl, 7-isoindolyl, 2-furyl, 3-furyl, 2-benzofuranyl, 3-benzofuranyl, 4-benzofuranyl, 5-benzofuranyl, 6-benzofuranyl, 7-benzofuranyl, 1-isobenzofuranyl, 3-isobenzofuranyl, 4-isobenzofuranyl, 5-isobenzofuranyl, 6-isobenzofuranyl, 7-isobenzofuranyl, quinolyl, 3-quinolyl, 4-quinolyl, 5-quinolyl, 6-quinolyl, 7-quinolyl, 8-quinolyl, 1-isoquinolyl, 3-isoquinolyl, 4-isoquinolyl, 5-isoquinolyl, 6-isoquinolyl, 7-isoquinolyl, 8-isoquinolyl, 2-quinoxalinyl, 5-quinoxalinyl, 6-quinoxalinyl, 1-carbazolyl, 2-carbazolyl, 3-carbazolyl, 4-carbazolyl, 9-carbazolyl, 1-phenanthridinyl, 2-phenanthridinyl, 3-phenanthridinyl, 4-phenanthridinyl, 6-phenanthridinyl, 7-phenanthridinyl, 8-phenanthridinyl, 9-phenanthridinyl, 10-phenanthryldinyl, 1-acridinyl, 2-acridinyl, 3-acridinyl, 4-acridinyl, 9-acridinyl, 1,7-phenanthroline-2-yl, 1,7-phenanthroline-3-yl, 1,7-phenanthroline-4-yl, 1,7-phenanthroline-5-yl, 1,7-phenanthroline-6-yl, 1,'7-phenanthroline-8-yl, 1,7-phenanthroline-9-yl, 1,7-phenanthroline-10-yl, 1,8-phenanthroline-2-yl, 1,8-phenanthroline-3-yl, 1,8-phenanthroline-4-yl, 1,8-phenanthroline-5-yl, 1,8-phenanthroline-6-yl, 1,8-phenanthroline-7-yl, 1,8-phenanthroline-9-yl, 1,8-phenanthroline-10-yl, 1,9-phenanthroline-2-yl, 1,9-phenanthroline-3-yl, 1,9-phenanthroline-4-yl, 1,9-phenanthroline-5-yl, 1,9-phenanthroline-6-yl, 1,9-phenanthroline-7-yl, 1,9-phenanthroline-8-yl, 1,9-phenanthroline-10-yl, 1,10-phenanthroline-2-yl, 1,10-phenanthroline-3-yl, 1,10-phenanthroline-4-yl, 1,10-phenanthroline-5-yl, 2,9-phenanthroline-1-yl, 2,9-phenanthroline-3-yl, 2,9-phenanthroline-4-yl, 2,9-phenanthroline-5-yl, 2,9-phenanthroline-6-yl, 2,9-phenanthroline-7-yl, 2,9-phenanthroline-8-yl, 2,9-phenanthroline-10-yl, 2,8-phenanthroline-1-yl, 2,8-phenanthroline-3-yl, 2,8-phenanthroline-4-yl, 2,8-phenanthroline-5-yl, 2,8-phenanthroline-6-yl, 2,8-phenanthroline-7-yl, 2,8-phenanthroline-9-yl, 2,8-phenanthroline-10-yl, 2,7-phenanthroline-1-yl, 2,7-phenanthroline-3-yl, 2,7-phenanthroline-4-yl, 2,7-phenanthroline-5-yl, 2,7-phenanthroline-6-yl, 2,7-phenanthroline-8-yl, 2,7-phenanthroline-9-yl, 2,7-phenanthroline-10-yl, 1-phenazinyl, 2-phenazinyl, 1-phenothiazinyl, 2-phenothiazinyl, 3-phenothiazinyl, 4-phenothiazinyl, 10-phenothiazinyl, 1-phenoxazinyl, 2-phenoxazinyl, 3-phenoxazinyl, 4-phenoxazinyl, 10-phenoxazinyl, 2-oxazolyl, 4-oxazolyl, 5-oxazolyl, 2-oxadiazolyl, 5-oxadiazolyl, 3-furazanyl, 2-thienyl, 3-thienyl, 2-methylpyrrole-1-yl, 2-methylpyrrole-3-yl, 2-methylpyrrole-4-yl, 2-methylpyrrole-5-yl, 3-methylpyrrole-1-yl, 3-methylpyrrole-2-yl, 3-methylpyrrole-4-yl, 3-methylpyrrole-5-yl, 2-t-butylpyrrole-4-yl, 3-(2-phenylpropyl)pyrrole-1-yl, 2-methyl-1-indolyl, 4-methyl-1-indolyl, 2-methyl-3-indolyl, 4-methyl-3-indolyl, 2-t-butyl-1-indolyl, 4-t-butyl-1-indolyl, 2-t-butyl-3-indolyl and 4-t-butyl-3-indolyl.

The examples of the substituted or non-substituted alkyl group include methyl, ethyl, propyl, isopropyl, n-butyl, s-butyl, isobutyl, t-butyl, n-pentyl, n-hexyl, n-heptyl, n-octyl, hydroxymethyl, 1-hydroxyethyl, 2-hydroxyethyl, 2-hydroxyisobutyl, 1,2-dihydroxyethyl, 1,3-dihydroxyisopropyl, 2,3-dihydroxy-t-butyl, 1,2,3-trihydroxypropyl, chloromethyl, 1-chloroethyl, 2-chloroethyl, 2-chloroisobutyl, 1,2-dichloroethyl, 1,3-dichloroisopropyl, 2,3-dichloro-t-butyl, 1,2,3-trichloropropyl, bromomethyl, 1-bromoethyl, 2-bromoethyl, 2-bromoisobutyl, 1,2-dibromoethyl, 1,3-dibromoisopropyl, 2,3-dibromo-t-butyl, 1,2,3-tribromopropyl, iodomethyl, 1-iodoethyl, 2-iodoethyl, 2-iodoisobutyl, 1,2-diiodoethyl, 1,3-diiodoisopropyl, 2,3-diiodo-t-butyl, 1,2,3-triiodopropyl, aminomethyl, 1-aminoethyl, 2-aminoethyl, 2-aminoisobutyl, 1,2-diaminoethyl, 1,3-diaminoisopropyl, 2,3-diamino-t-butyl, 1,2,3-triaminopropyl, cyanomethyl, 1-cyanoethyl, 2-cyanoethyl, 2-cyanoisobutyl, 1,2-dicyanoethyl, 1,3-dicyanoisopropyl, 2,3-dicyano-t-butyl, 1,2,3-tricyanopropyl, nitromethyl, 1-nitroethyl, 2-nitroethyl, 2-nitroisobutyl, 1,2-dinitroethyl, 1,3-dinitroisopropyl, 2,3-dinitro-t-butyl, 1,2,3-trinitropropyl, cyclopropyl, cyclobutyl, cyclopentyl, cyclohexyl, 4-methylcyclohexyl, 1-adamantyl, 2-adamantyl, 1-norbornyl and 2-norbornyl.

The substituted or non-substituted alkoxy group is a group represented by -OY, and the examples of Y include methyl, ethyl propyl, isopropyl, n-butyl, s-butyl, isobutyl, t-butyl, n-pentyl, n-hexyl, n-heptyl, n-octyl, hydroxymethyl, 1-hydroxyethyl, 2-hydroxyethyl, 2-hydroxyisobutyl, 1,2-dihydroxyethyl, 1,3-dihydroxyisopropyl, 2,3-dihydroxy-t-butyl, 1,2,3-trihydroxypropyl, chloromethyl, 1-chloroethyl, 2-chloroethyl, 2-chloroisobutyl, 1,2-dichloroethyl, 1,3-dichloroisopropyl, 2.3-dichloro-t-butyl, 1,2,3-trichloropropyl, bromomethyl, 1-bromoethyl, 2-bromoethyl, 2-bromoisobutyl, 1,2-dibromoethyl, 1,3-dibromoisopropyl, 2,3-dibromo-t-butyl, 1,2,3-tribromopropyl, iodomethyl, 1-iodoethyl, 2-iodoethyl, 2-iodoisobutyl, 1,2-diiodoethyl, 1,3-diiodoisopropyl, 2,3-diiodo-t-butyl, 1,2,3-triiodopropyl, aminomethyl, 1-aminoethyl, 2-aminoethyl, 2-aminoisobutyl, 1,2-diaminoethyl, 1,3-diaminoisopropyl, 2,3-diamino-t-butyl, 1,2,3-triaminopropyl, cyanomethyl, 1-cyanoethyl, 2-cyanoethyl, 2-cyanoisobutyl, 1,2-dicyanoethyl, 1,3-dicyanoisopropyl, 2,3-dicyano-t-butyl, 1,2,3-tricyanopropyl, nitromethyl, 1-nitroethyl, 2-nitroethyl, 2-nitroisobutyl, 1,2-dinitroethyl, 1,3-dinitroisopropyl, 2,3-dinitro-t-butyl and 1,2,3-trinitropropyl.

The examples of the substituted or non-substituted aralkyl group include benzyl, 1-phenylethyl, 2-phenylethyl, 1-phenylisopropyl, 2-phenylisopropyl, phenyl-t-butyl, α-naphthylmethyl, 1-α-naphthylethyl, 2-α-naphthylethyl, 1-α-naphthylisopropyl, 2-α-naphthylisopropyl, β-naphthylmethyl, 1-β-naphthylethyl, 2-β-naphthylethyl, 1-β-naphthylisopropyl, 2-β-naphthylisopropyl, 1-pyrrolylmethyl, 2-(1-pyrrolyl)ethyl, p-methylbenzyl, m-methylbenzyl, o-methylbenzyl, p-chlorobenzyl, m-chlorobenzyl, o-chlorobenzyl, p-bromobenzyl, m-bromobenzyl, o-bromobenzyl, p-iodobenzyl, m-iodobenzyl, o-iodobenzyl, p-hydroxybenzyl, m-hydroxybenzyl, o-hydroxybenzyl, p-aminobenzyl, m-aminobenzyl, o-aminobenzyl, p-nitrobenzyl, m-nitrobenzyl, o-nitrobenzyl, p-cyanobenzyl, m-cyanobenzyl, o-cyanobenzyl, 1-hydroxy-2-phenylisopropyl and 1-chloro-2-phenylisopropyl.

The substituted or non-substituted aryloxy group is a group represented by -OY', and the examples of Y' include phenyl, 1-naphthyl, 2-naphthyl, 1-anthryl, 2-anthryl, 9-anthryl, 1-phenanthryl, 2-phenanthryl, 3-phenanthryl, 4-phenanthryl, 9-phenanthryl, 1-naphthacenyl, 2-naphthacenyl, 9-naphthacenyl, 1-pyrenyl, 2-pyrenyl, 4-pyrenyl, 2-biphenylyl, 3-biphenylyl, 4-biphenylyl, p-terphenyl-4-yl, p-terphenyl-3-yl, p-terphenyl-2-yl, m-terphenyl-4-yl, m-terphenyl-3-yl, m-terphenyl-2-yl, o-tolyl, m-tolyl, p-tolyl, p-t-butylphenyl, p-(2-phenylpropyl)phenyl, 3-methyl-2-naphthyl, 4-methyl-1-naphthyl, 4-methyl-1-anthryl, 4'-methylbiphenylyl, 4"-t-butyl-p-terphenyl-4-yl, 2-pyrrolyl, 3-pyrrolyl, pyrazinyl, 2-pyridinyl, 3- pyridinyl, 4- pyridinyl, 2-indolyl, 3-indolyl, 4-indolyl, 5-indonyl, 6-indolyl, 7-indolyl, 1-isoindolyl, 3-isoindolyl, 4-isoindolyl, 5-isoindolyl, 6-isoindolyl, 7-isoindolyl, 2-furyl, 3-furyl, 2-benzofuranyl, 3-benzofuranyl, 4-benzofuranyl, 5-benzofuranyl, 6-benzofuranyl, 7-benzofuranyl, 1-isobenzofuranyl, 3-isobenzofuranyl, 4-isobenzofuranyl, 5-isobenzofuranyl, 6-isobenzofuranyl, 7-isobenzofuranyl, 2-quinolyl, 3-quinolyl, 4-quinolyl, 5-quinolyl, 6-quinolyl, 7-quinolyl, 8-quinolyl, 1-isoquinolyl, 3-isoquinolyl, 4-isoquinolyl, 5-isoquinolyl, 6-isoquinolyl, 7-isoquinolyl, 8-isoquinolyl, 2-quinoxalinyl, 5-quinoxalinyl, 6-quinoxalinyl, 1-carbazolyl, 2-carbazolyl, 3-carbazolyl, 4-carbazolyl, 1-phenanthridinyl, 2-phenanthridinyl, 3-phenanthridinyl, 4-phenanthridinyl, 6-phenanthridinyl, 7-phenanthridinyl, 8-phenanthridinyl, 9-phenanthridinyl, 10-phenanthridinyl, 1-acridinyl, 2-acridinyl, 3-acridinyl, 4-acridinyl, 9-acridinyl, 1,7-phenanthroline-2-yl, 1,7-phenanthroline-3-yl, 1,7-phenanthroline-4-yl, 1,7-phenanthroline-5-yl, 1,7-phenanthroline-6-yl, 1,7-phenanthroline-8-yl, 1,7-phenanthroline-9-yl, 1,7-phenanthroline-10-yl, 1,8-phenanthroline-2-yl, 1,8-phenanthroline-3-yl, 1,8-phenanthroline-4-yl, 1,8-phenanthroline-5-yl, 1,8-phenanthroline-6-yl, 1,8-phenanthroline-7-yl, 1,8-phenanthroline-9-yl, 1,8-phenanthroline-10-yl, 1,9-phenanthroline-2-yl, 1,9-phenanthroline-3-yl, 1,9-phenanthroline-4-yl, 1,9-phenanthroline-5-yl, 1,9-phenanthroline-6-yl, 1,9-phenanthroline-7-yl, 1,9-phenanthroline-8-yl, 1,9-phenanthroline-10-yl, 1,10-phenanthroline-2-yl, 1,10-phenanthroline-3-yl, 1,10-phenanthroline-4-yl, 1,10-phenanthroline-5-yl, 2,9-phenanthroline-1-yl, 2,9-phenanthroline-3-yl, 2,9-phenanthroline-4-yl, 2,9-phenanthroline-5-yl, 2,9-phenanthroline-6-yl, 2,9-phenanthroline-7-yl, 2,9-phenanthroline-8-yl, 2,9-phenanthroline-10-yl, 2,8-phenanthroline-1-yl, 2,8-phenanthroline-3-yl, 2,8-phenanthroline-4-yl, 2,8-phenanthroline-5-yl, 2,8-phenanthroline-6-yl, 2,8-phenanthroline-7-yl, 2,8-phenanthroline-9-yl, 2,8-phenanthroline-10-yl, 2,7-phenanthroline-1-yl, 2,7-phenanthroline-3-yl, 2,7-phenanthroline-4-yl, 2,7-phenanthroline-5-yl, 2,7-phenanthroline-6-yl, 2,7-phenanthroline-8-yl, 2,7-phenanthroline-9-yl, 2,7-phenanthroline-10-yl, 1-phenazinyl, 2-phenazinyl, 1-phenothiazinyl, 3-phenothiazinyl, 4-phenothiazinyl, 1-phenoxazinyl, 2-phenoxazinyl, 3-phenoxazinyl, 4-phenoxazinyl, 2-oxazolyl, 4-oxazolyl, 5-oxazolyl, 2-oxadiazolyl, 5-oxadiazolyl, 3-furazanyl?, 2-thienyl, 3-thienyl, 2-methylpyrrole-1-yl, 2-methylpyrrole-3-yl, 2-methylpyrrole-4-yl, 2-methylpyrrole-5-yl, 3-methylpyrrole-1-yl, 3-methylpyrrole-2-yl, 3-methylpyrrole-4-yl, 3-methylpyrrole-5-yl, 2-t-butylpyrrole-4-yl, 3-(2-phenylpropyl)pyrrole-1-yl, 2-methyl-1-indolyl, 4-methyl-1-indolyl, 2-methyl-3-indolyl, 4-methyl-3-indolyl, 2-t-butyl-1-indolyl, 4-t-butyl-1-indolyl, 2-t-butyl-3-indolyl and 4-t-butyl-3-indolyl.

The substituted or non-substituted arylthio group is a group represented by -SY", and the examples of Y" include phenyl, 1-naphthyl, 2-naphthyl, 1-anthryl, 2-anthryl, 9-anthryl, 1-phenanthryl, 2-phenanthryl, 3-phenanthryl, 4-phenanthryl, 9-phenanthryl, 1-naphthacenyl, 2-naphthacenyl, 9-naphthacenyl, 1-pyrenyl, 2-pyrenyl, 4-pyrenyl, 2-biphenylyl, 3-biphenylyl, 4-biphenylyl, p-terphenyl-4-yl, p-terphenyl-3-yl, p-terphenyl-2-yl, m-terphenyl-4-yl, m-terphenyl-3-yl, m-terphenyl-2-yl, o-tolyl, m-tolyl, p-tolyl, p-t-butylphenyl, p-(2-phenylpropyl)phenyl, 3-methyl-2-naphthyl, 4-methyl-1-naphthyl, 4-methyl-1-anthryl, 4'-methylbiphenylyl, 4"-t-butyl-p-terphenyl-4-yl, 2-pyrrolyl, 3-pyrrolyl, pyrazinyl, 2-pyridinyl, 3- pyridinyl, 4- pyridinyl, 2-indolyl, 3-indolyl, 4-indolyl, 5-indolyl, 6-indolyl, 7-indolyl, 1-isoindolyl, 3-isoindolyl, 4-isoindolyl, 5-isoindolyl, 6-isoindolyl, 7-isoindolyl, 2-furyl, 3-furyl, 2-benzofuranyl, 3-benzofuranyl, 4-benzofuranyl, 5-benzofuranyl, 6-benzofuranyl, 7-benzofuranyl, 1-isobenzofuranyl, 3-isobenzofuranyl, 4-isobenzofuranyl, 5-isobenzofuranyl, 6-isobenzofuranyl, 7-isobenzofuranyl, 2-quinolyl, 3-quinolyl, 4-quinolyl, 5-quinolyl, 6-quinolyl, 7-quinolyl, 8-quinolyl, 1-isoquinolyl, 3-isoquinolyl, 4-isoquinolyl, 5-isoquinolyl, 6-isoquinolyl, 7-isoquinolyl, 8-isoquinolyl, 2-quinoxalinyl, 5-quinoxalinyl, 6-quinoxalinyl, 1-carbazolyl, 2-carbazolyl, 3-carbazolyl, 4-carbazolyl, 1-phenanthridinyl, 2-phenanthridinyl, 3-phenanthridinyl, 4-phenanthridinyl, 6-phenanthridinyl, 7-phenanthridinyl, 8-phenanthridinyl, 9-phenanthridinyl, 10-phenanthridinyl, 1-acridinyl, 2-acridinyl, 3-acridinyl, 4-acridinyl, 9-acridinyl, 1,7-phenanthroline-2-yl, 1,7-phenanthroline-3-yl, 1,7-phenanthroline-4-yl, 1,7-phenanthroline-5-yl, 1,7-phenanthroline-6-yl, 1,7-phenanthroline-8-yl, 1,7-phenanthroline-9-yl, 1,7-phenanthroline-10-yl, 1,8-phenanthroline-2-yl, 1,8-phenanthroline-3-yl, 1,8-phenanthroline-4-yl, 1,8-phenanthroline-5-yl, 1,8-phenanthroline-6-yl, 1,8-phenanthroline-7-yl, 1,8-phenanthroline-9-yl, 1,8-phenanthroline-10-yl, 1,9-phenanthroline-2-yl, 1,9-phenanthroline-3-yl, 1,9-phenanthroline-4-yl, 1,9-phenanthroline-5-yl, 1,9-phenanthroline-6-yl, 1,9-phenanthroline-7-yl, 1,9-phenanthroline-8-yl, 1,9-phenanthroline-10-yl, 1,10-phenanthroline-2-yl, 1,10-phenanthroline-3-yl, 1,10-phenanthroline-4-yl, 1,10-phenanthroline-5-yl, 2,9-phenanthroline-1-yl, 2,9-phenanthroline-3-yl, 2,9-phenanthroline-4-yl, 2,9-phenanthroline-5-yl, 2,9-phenanthroline-6-yl, 2,9-phenanthroline-7-yl, 2,9-phenanthroline-8-yl, 2,9-phenanthroline-10-yl, 2,8-phenanthroline-1-yl, 2,8-phenanthroline-3-yl, 2,8-phenanthroline-4-yl, 2,8-phenanthroline-5-yl, 2,8-phenanthroline-6-yl, 2,8-phenanthroline-7-yl, 2,8-phenanthroline-9-yl, 2,8-phenanthroline-10-yl, 2,7-phenanthroline-1-yl, 2,7-phenanthroline-3-yl, 2,7-phenanthroline-4-yl, 2,7-phenanthroline-5-yl, 2,7-phenanthroline-6-yl, 2,7-phenanthroline-8-yl, 2,7-phenanthroline-9-yl, 2,7-phenanthroline-10-yl, 1-phenazinyl, 2-phenazinyl, 1-phenothiazinyl, 3-phenothiazinyl, 4-phenothiazinyl, 1-phenoxazinyl, 2-phenoxazinyl, 3-phenoxazinyl, 4-phenoxazinyl, 2-oxazolyl, 4-oxazolyl, 5-oxazolyl, 2-oxadiazolyl, 5-oxadiazolyl, 3-furazanyl, 2-thienyl, 3-thienyl, 2-methylpyrrole-1-yl, 2-methylpyrrole-3-yl, 2-methylpyrrole-4-yl, 2-methylpyrrole-5-yl, 3-methylpyrrole-1-yl, 3-methylpyrrole-2-yl, 3-methylpyrrole-4-yl, 3-methylpyrrole-5-yl, 2-t-butylpyrrole-4-yl, 3-(2-phenylpropyl)pyrrole-1-yl, 2-methyl-1-indolyl, 4-methyl-1-indolyl, 2-methyl-3-indolyl, 4-methyl-3-indolyl, 2-t-butyl-1-indolyl, 4-t-butyl-1-indolyl, 2-t-butyl-3-indolyl and 4-t-butyl-3-indolyl.

The substituted or non-substituted alkoxylcarbonyl group is a group represented by -COOZ, and the examples of Z include methyl, ethyl, propyl, isopropyl, n-butyl, s-butyl, isobutyl, t-butyl, n-pentyl, n-hexyl, n-heptyl, n-octyl, hydroxymethyl, 1-hydroxyethyl, 2-hydroxyethyl, 2-hydroxyisobutyl, 1,2-dihydroxyethyl, 1,3-dihydroxyisopropyl, 2,3-dihydroxy-t-butyl, 1,2,3-trihydroxypropyl, chloromethyl, 1-chloroethyl, 2-chloroethyl, 2-chloroisobutyl, 1,2-dichloroethyl, 1,3-dichloroisopropyl, 2,3-dichloro-t-butyl, 1,2,3-trichloropropyl, bromomethyl, 1-bromoethyl, 2-bromoethyl, 2-bromoisobutyl, 1,2-dibromoethyl, 1,3-dibromoisopropyl, 2,3-dibromo-t-butyl, 1,2,3-tribromopropyl, iodomethyl, 1-iodoethyl, 2-iodoethyl, 2-iodoisobutyl, 1,2-diiodoethyl, 1,3-diiodoisopropyl, 2,3-diiodo-t-butyl, 1,2,3-triiodopropyl, aminomethyl, 1-aminoethyl, 2-aminoethyl, 2-aminoisobutyl, 1,2-diaminoethyl, 1,3-diaminaisopropyl, 2,3-diamino-t-butyl, 1,2,3-triaminopropyl, cyanomethyl, 1-cyanoethyl, 2-cyanoethyl, 2-cyanoisobutyl, 1,2-dicyanoethyl, 1,3-dicyanoisopropyl, 2,3-dicyano-t-butyl, 1,2,3-tricyanopropyl, nitromethyl, 1-nitroethyl, 2-nitroethyl, 2-nitroisobutyl, 1,2-dinitroethyl, 1,3-dinitroisopropyl, 2,3-dinitro-t-butyl and 1,2,3-trinitropropyl.

The halogen atom includes fluorine, chlorine, bromine and iodine.

Provided that the aromatic amine compound of the present invention has to satisfy the conditions of (1) and/or (2):
(1) at least one of Ar³ to Ar⁶ is a substituted or non-substituted condensed aryl group having 10 to 50 nuclear carbon atoms and
(2) at least one of Ar¹ to Ar² is a substituted or non-substituted condensed aryl group having 12 to 50 nuclear carbon atoms.

The structure of the aromatic amine compound becomes complicated by satisfying the above conditions, and therefore it is less liable to be decomposed and improved in a durability of the compound itself.

The specific examples of the aromatic amine compound of the present invention represented by Formula (1) shall be shown below, but it shall not be restricted to the following exemplified compounds. In the following compounds, Me shows methyl; Et shows ethyl; tBu shows t-butyl; and iPr shows isopropyl.

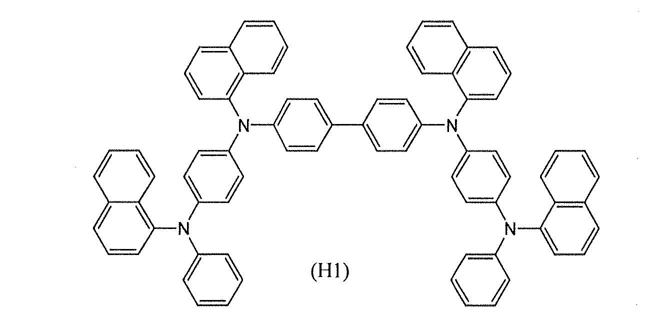

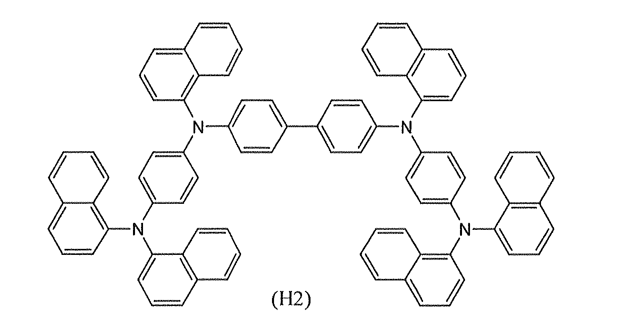

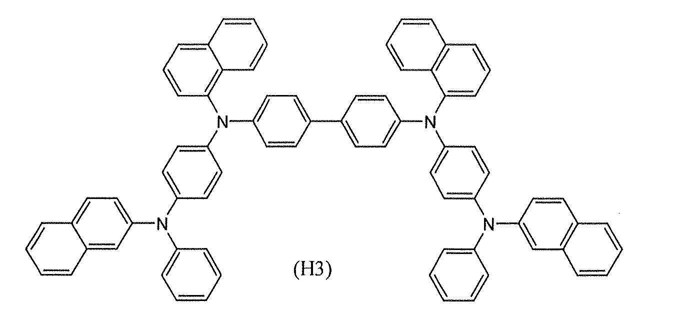

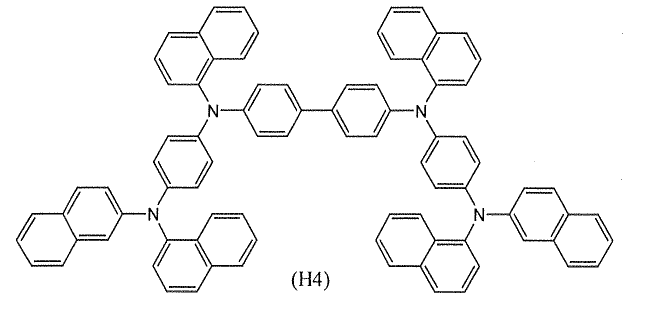

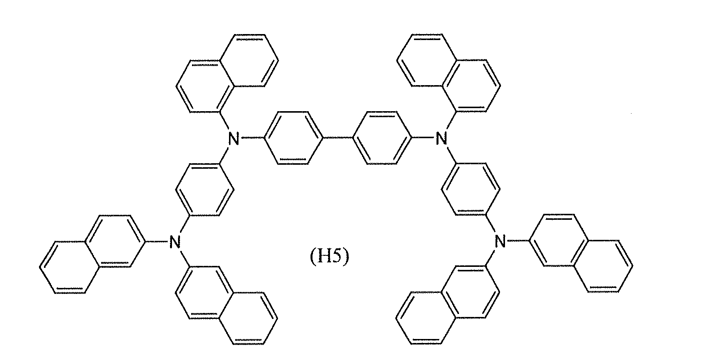

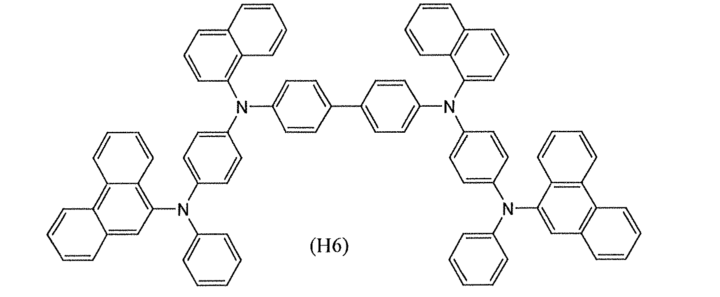

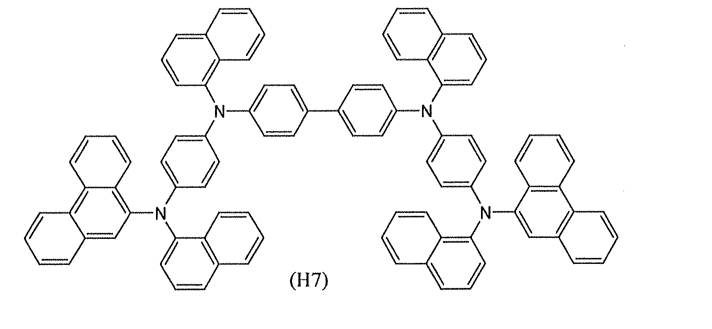

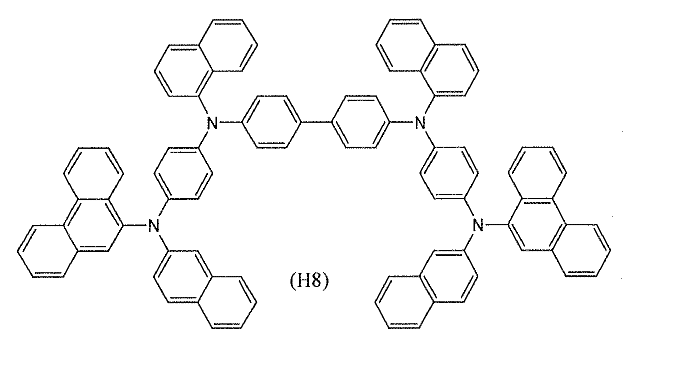

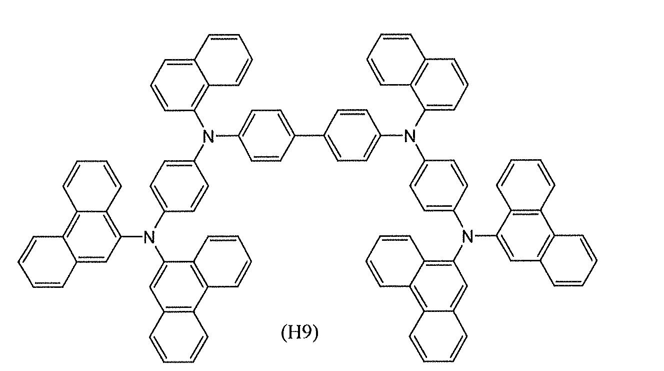

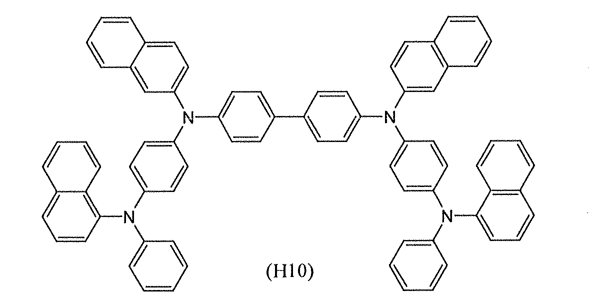

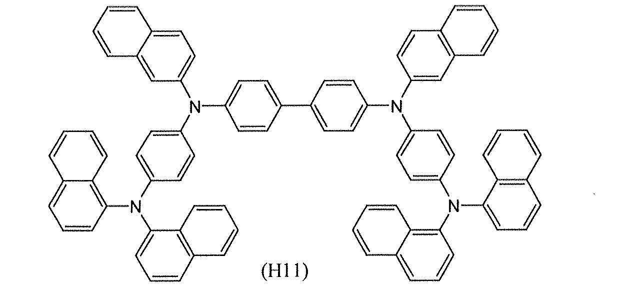

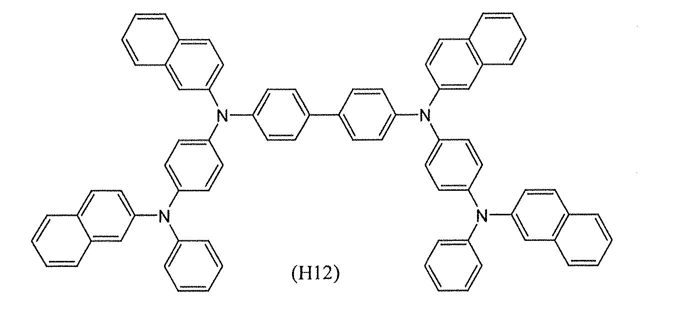

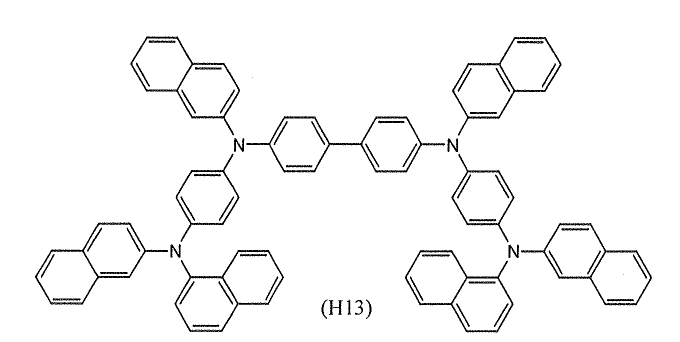

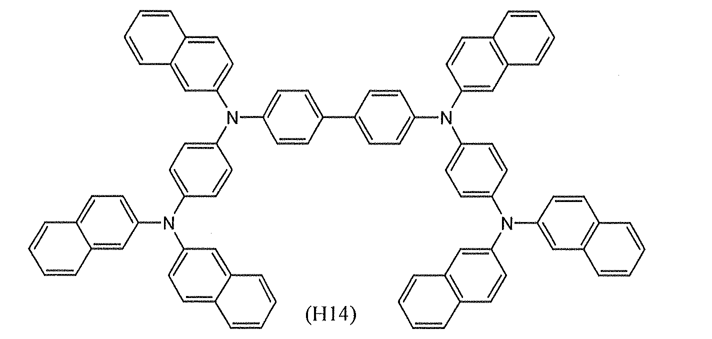

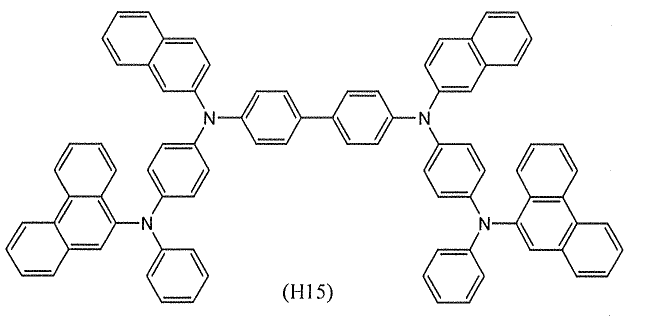

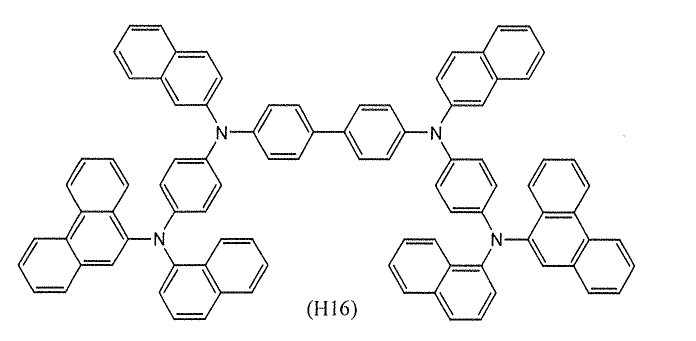

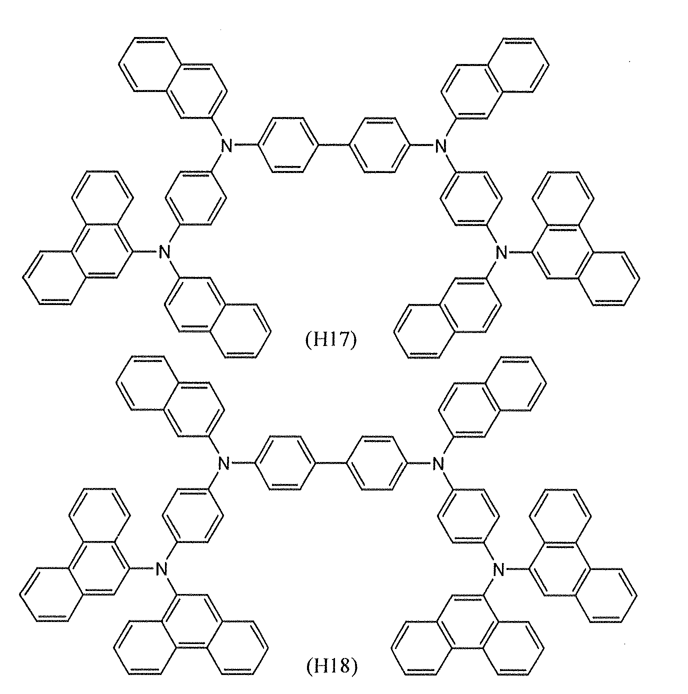

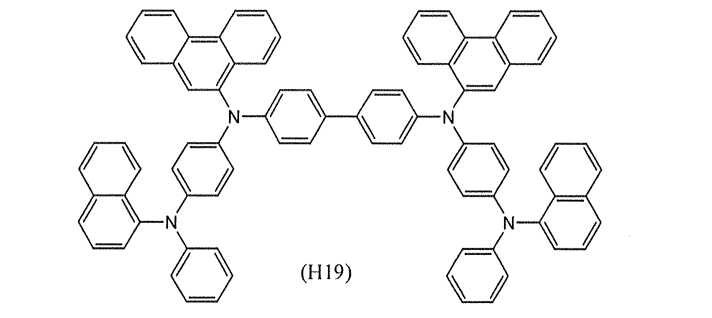

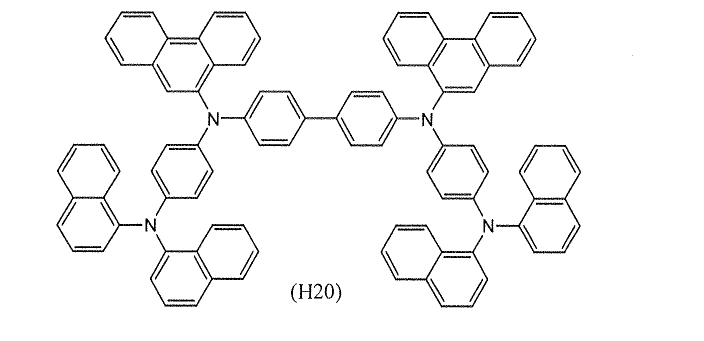

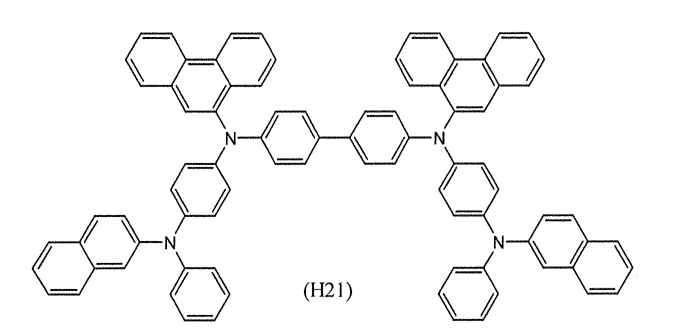

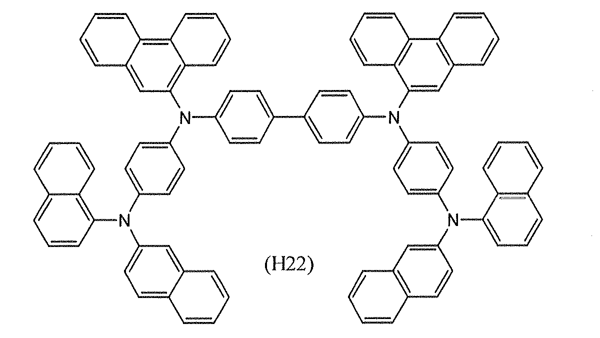

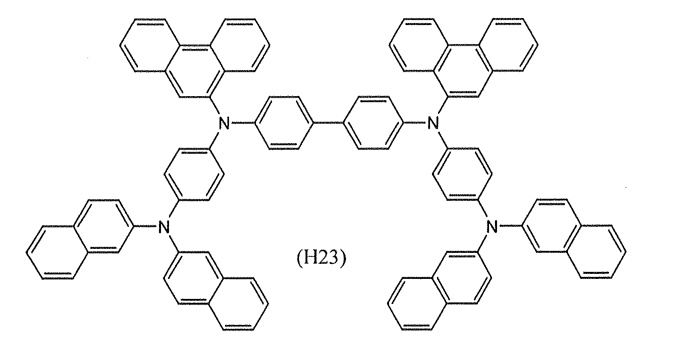

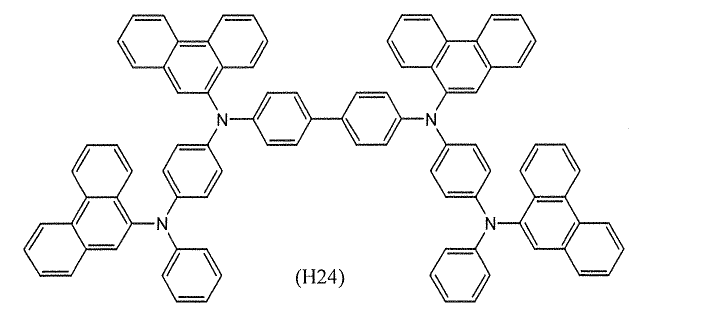

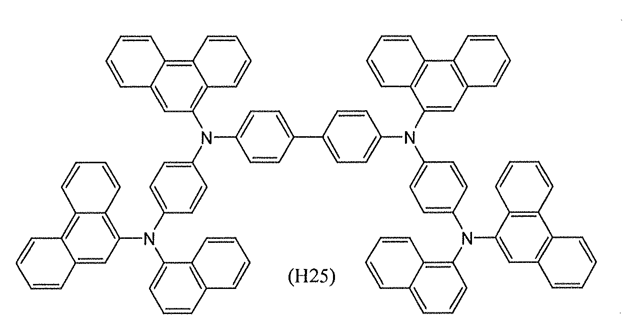

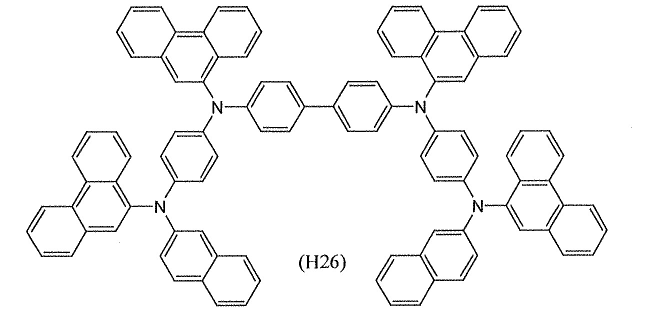

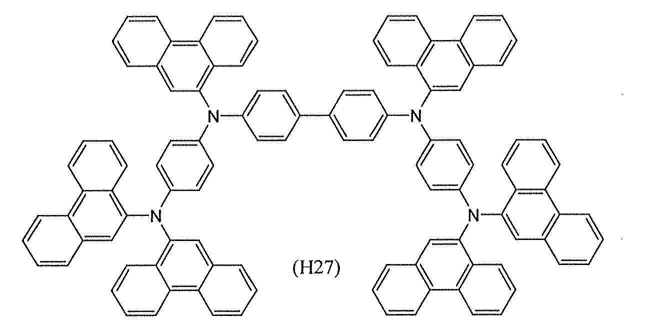

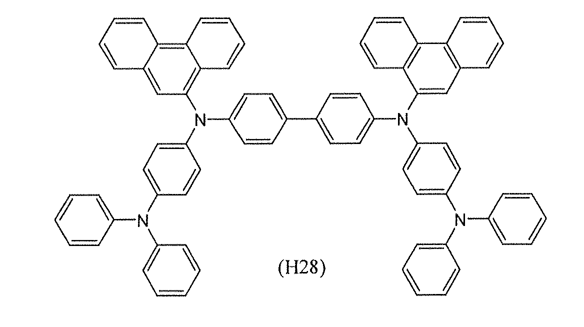

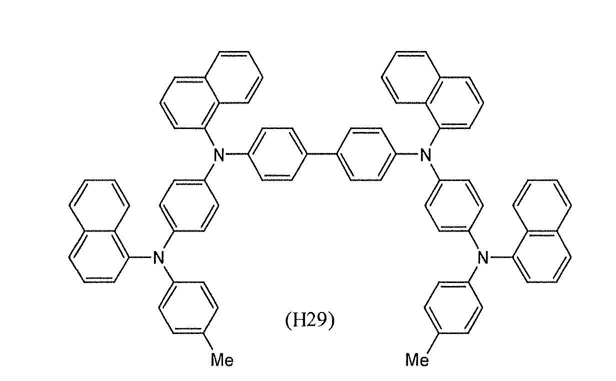

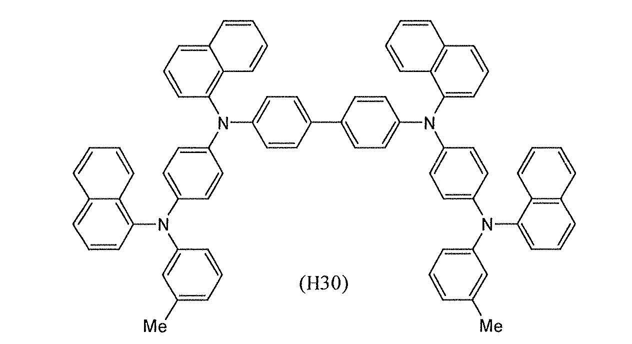

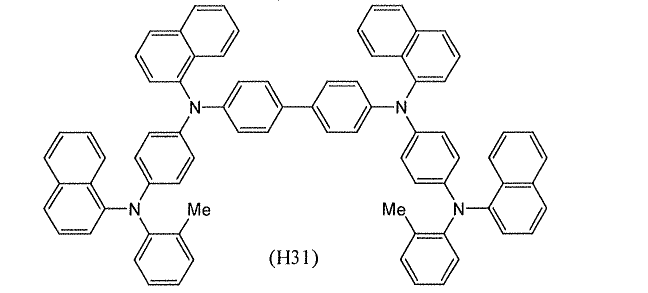

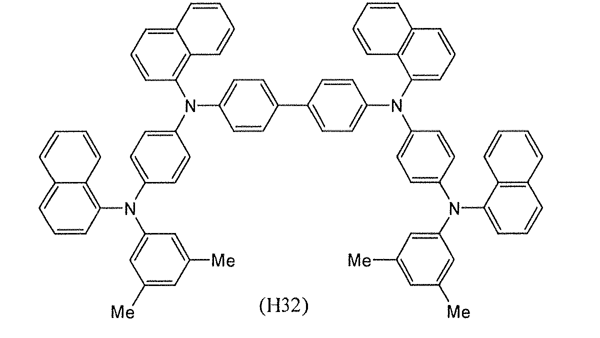

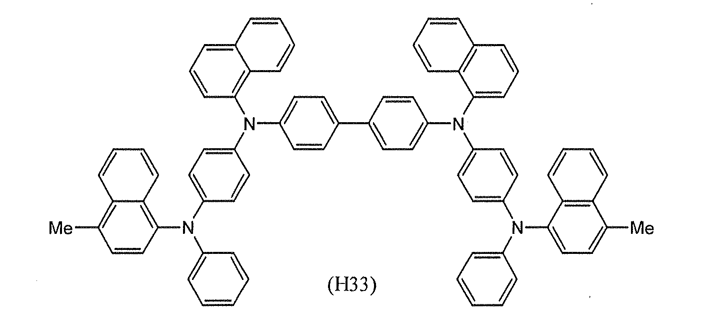

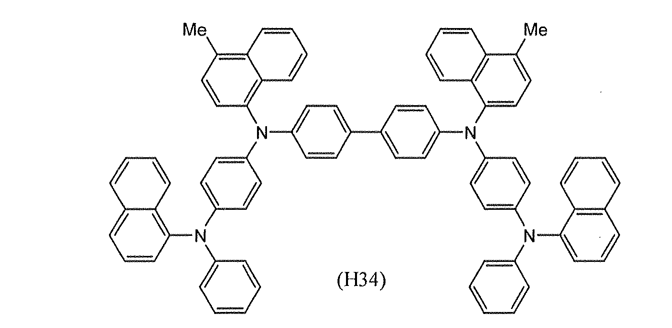

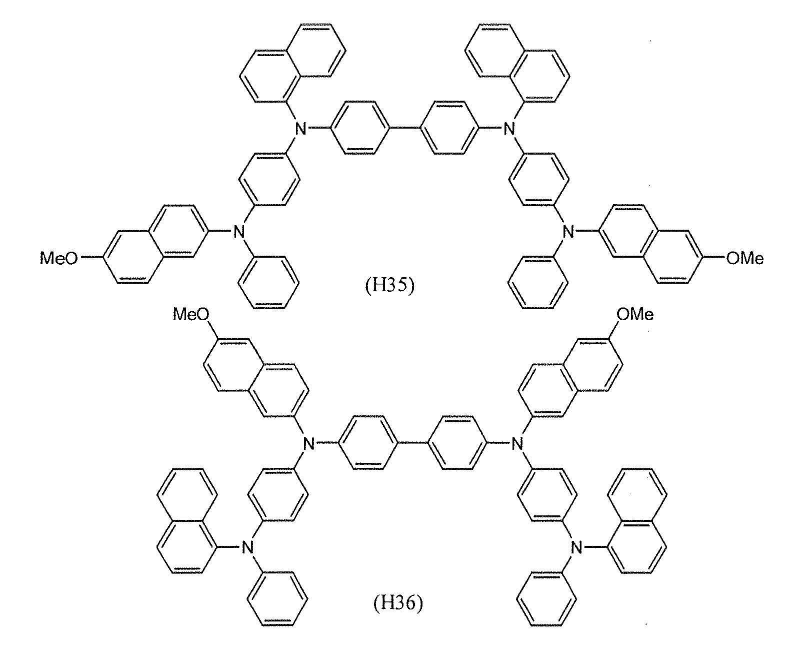

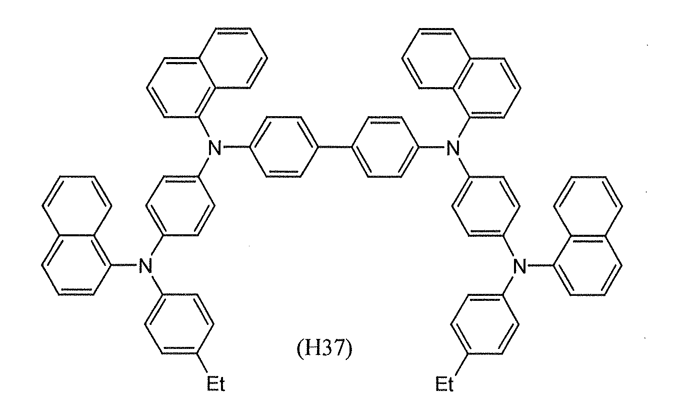

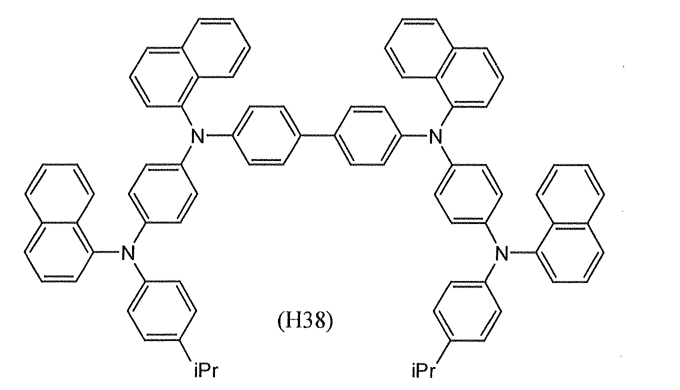

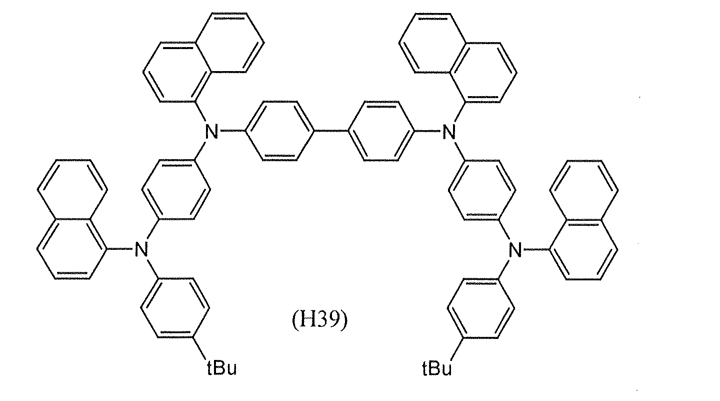

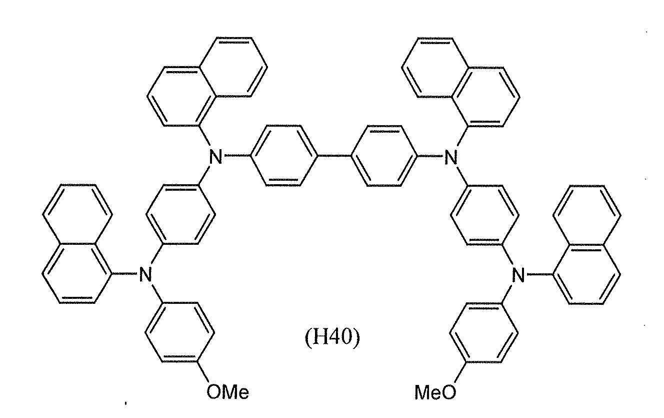

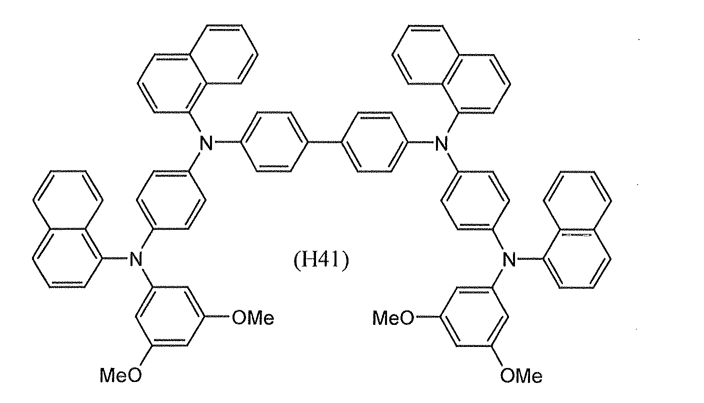

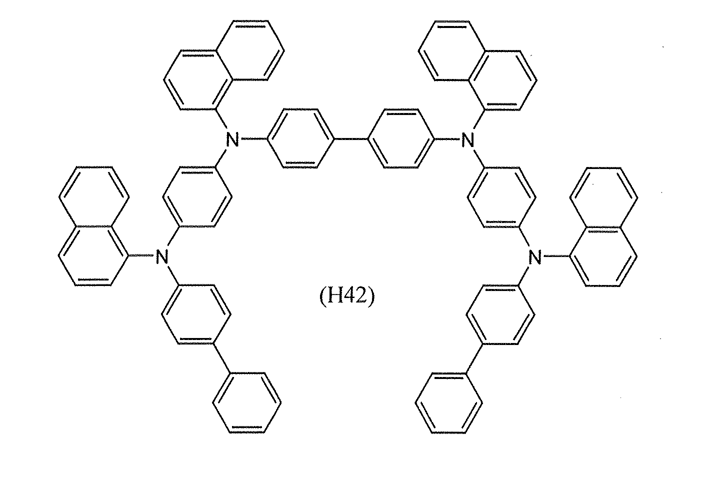

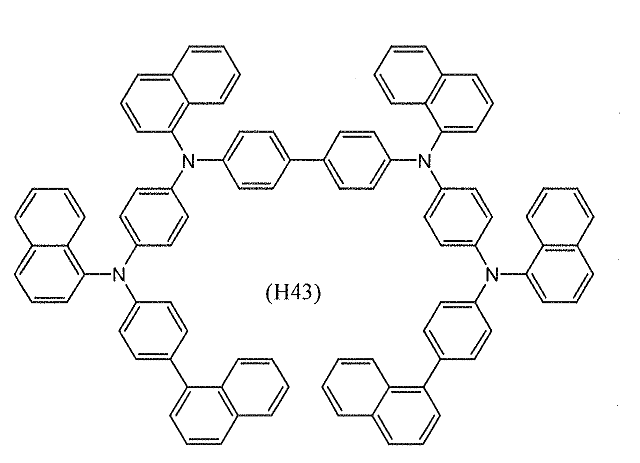

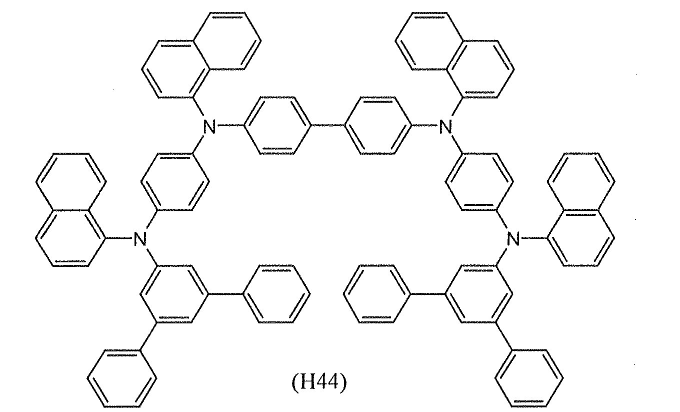

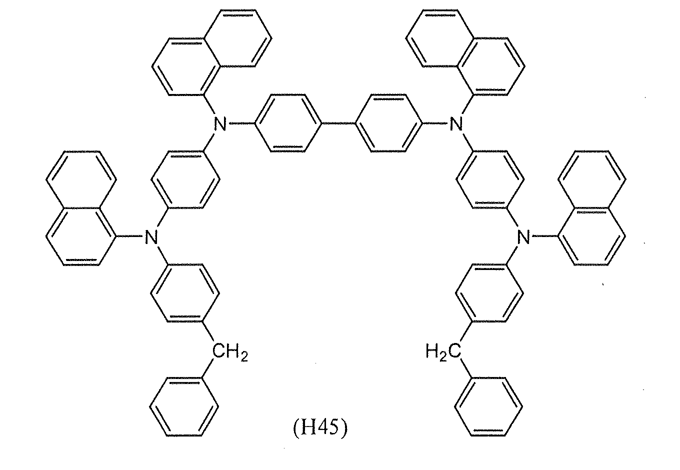

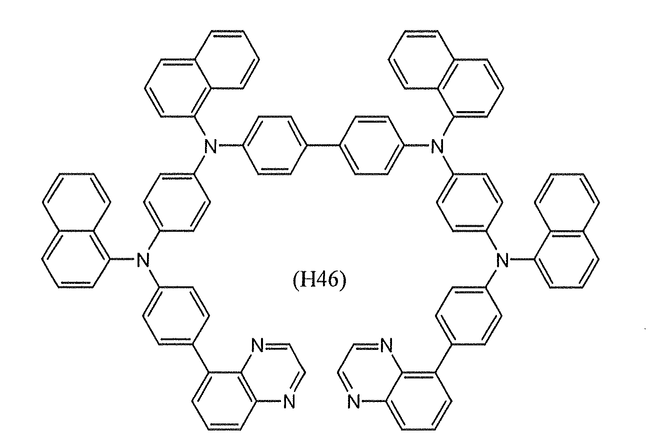

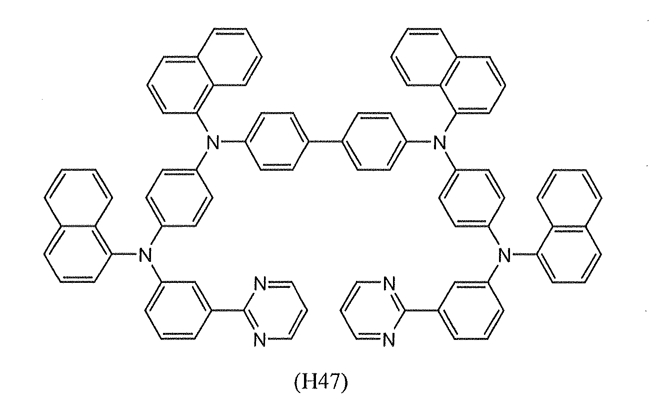

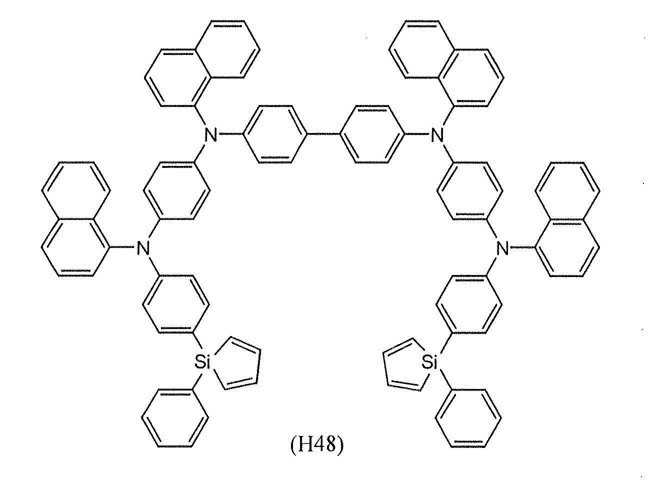

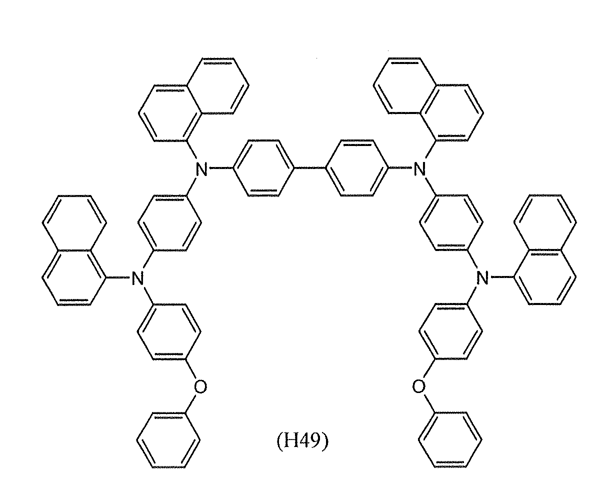

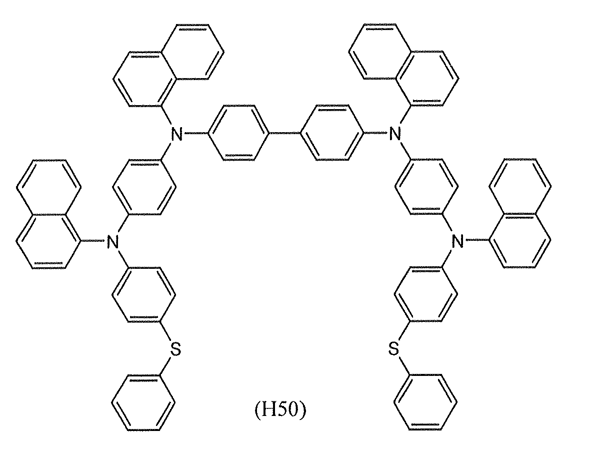

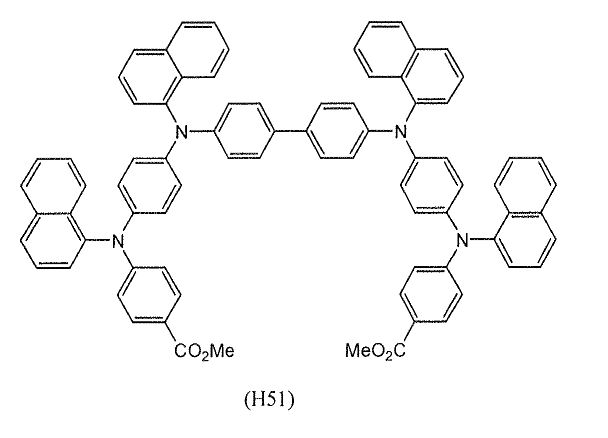

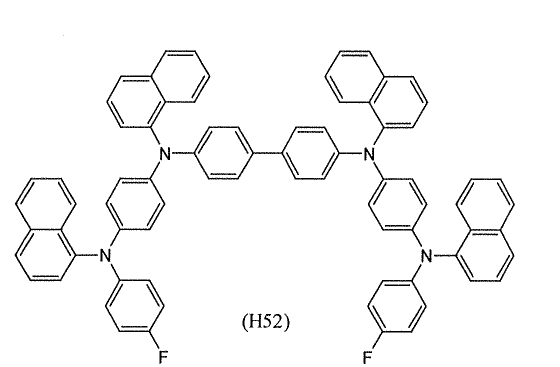

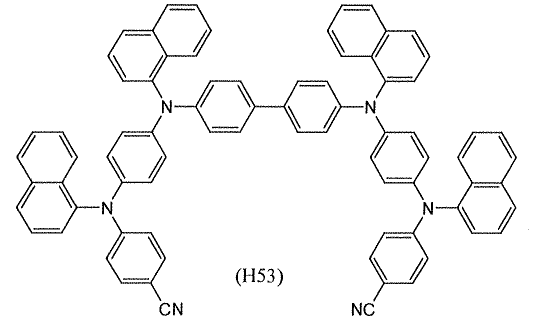

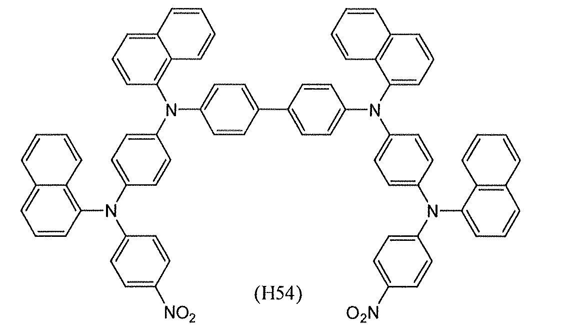

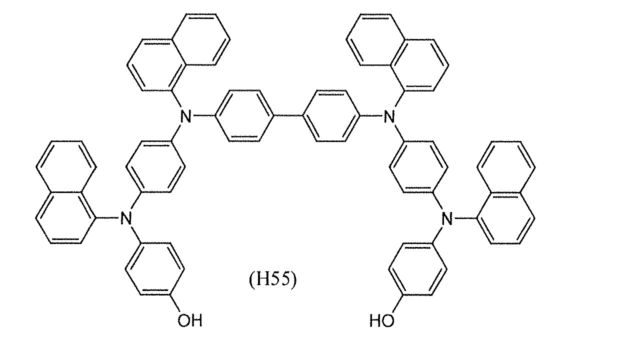

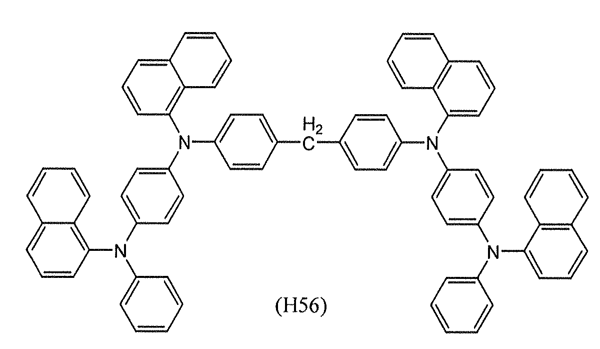

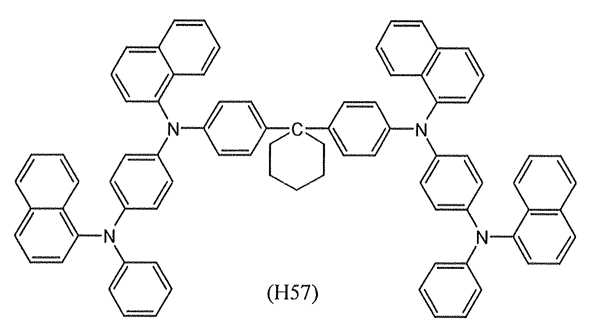

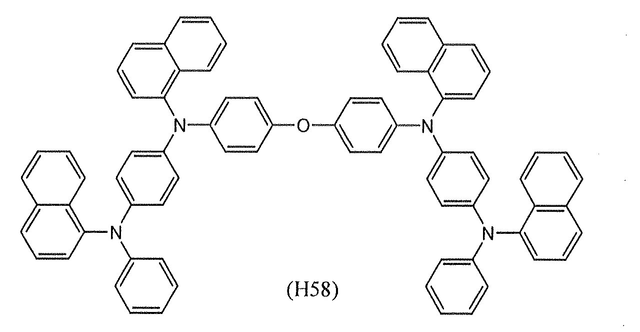

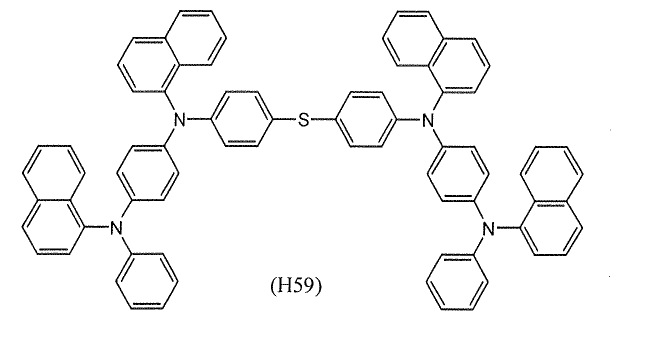

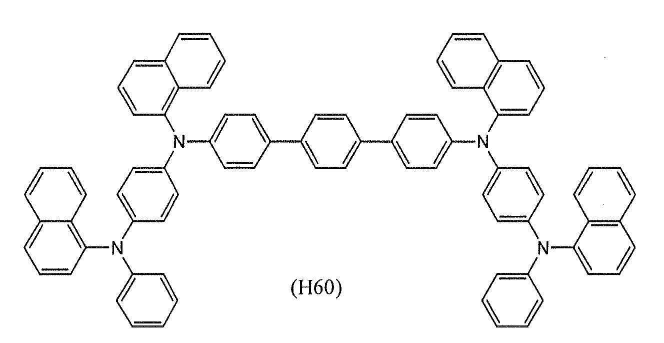

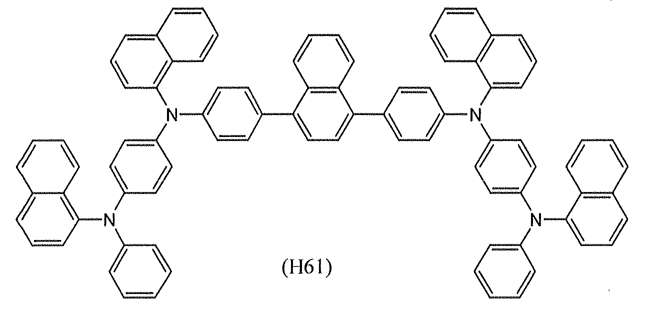

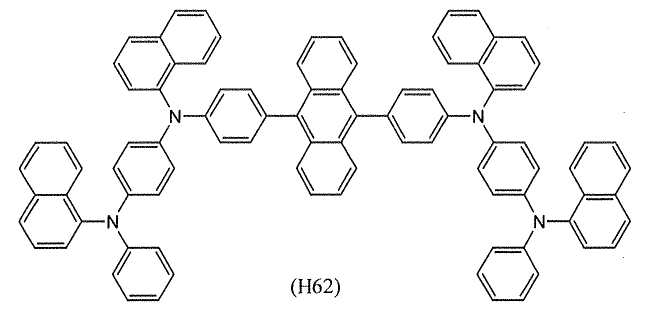

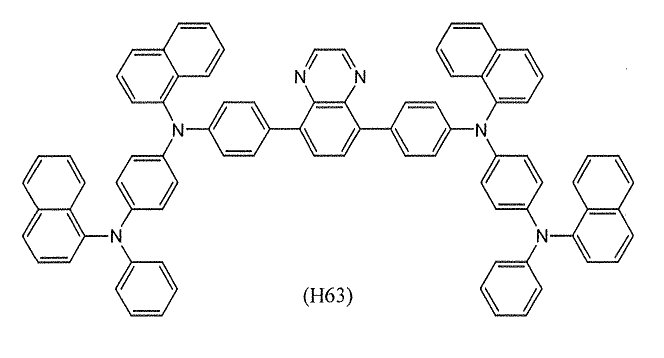

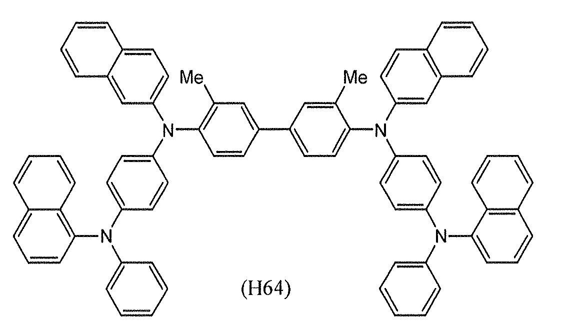

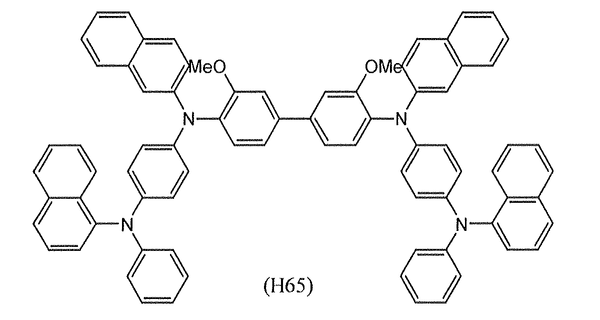

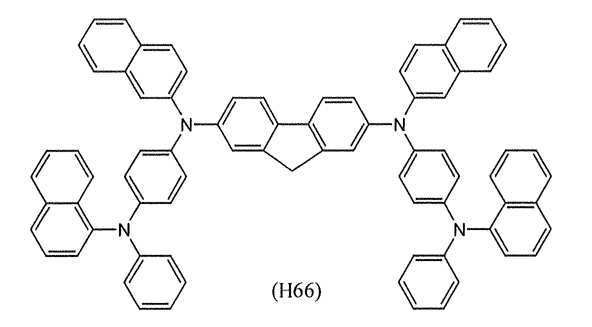

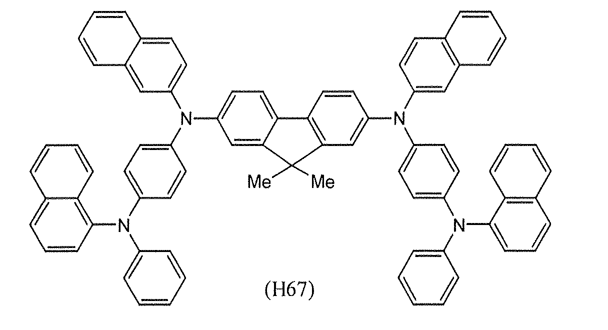

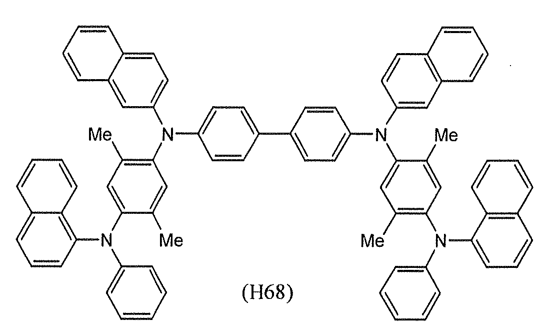

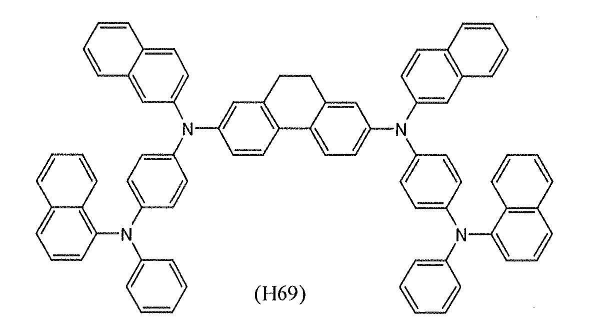

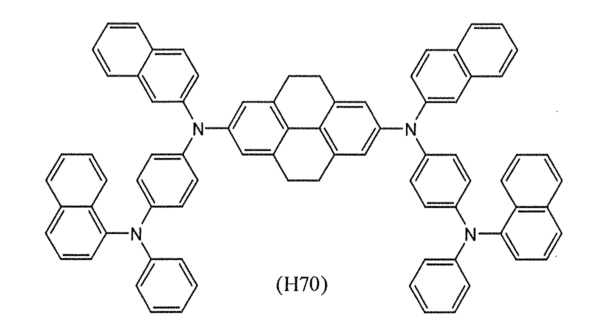

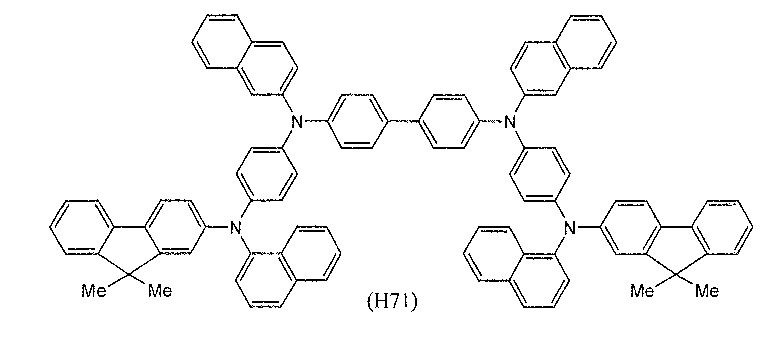

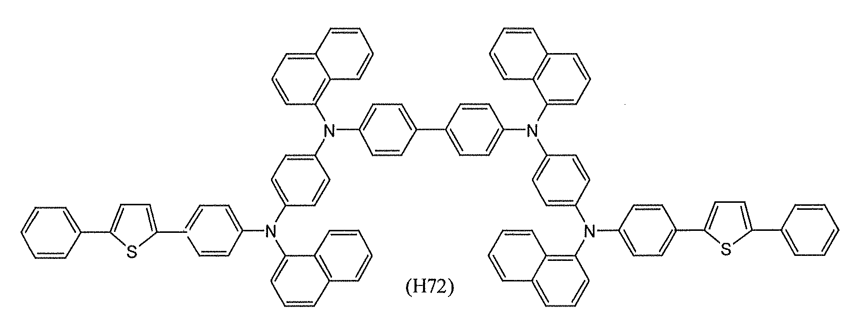

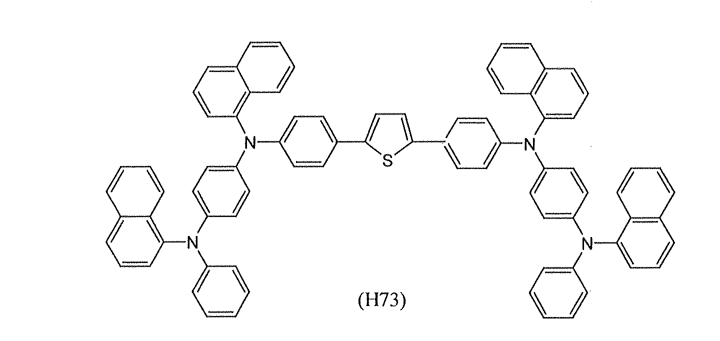

Next, the organic EL element of the present invention shall be explained.

In the organic EL element of the present invention in which an organic thin film layer comprising a single layer or plural layers including at least a light emitting layer is interposed between a cathode and an anode, at least one layer of the above organic thin film layers contains the aromatic amine derivative described above as a single component or a mixed component.

In the organic EL element of the present invention, it is preferred that the organic thin film layer described above has a hole transporting zone and that the above hole transporting zone contains the aromatic amine derivative of the present invention as a single component or a mixed component. Also, it is more preferred that the organic thin film layer described above has a hole injecting layer and that the above hole injecting layer contains the aromatic amine derivative of the present invention as a single component or a mixed component.

The element structure of the organic EL element of the present invention shall be explained below.

### (1) Structure of the organic EL element

The typical examples of the element structure of the organic EL element of the present invention include:
(1) Anode/light emitting layer/cathode
(2) Anode/hole injecting layer/light emitting layer/cathode
(3) Anode/light emitting layer/electron injecting layer/cathode
(4) Anode/hole injecting layer/light emitting layer/electron injecting layer/cathode
(5) Anode/organic semiconductor layer/light emitting layer/cathode
(6) Anode/organic semiconductor layer/electron barrier layer/light emitting layer/cathode
(7) Anode/organic semiconductor layer/light emitting layer/adhesion improving layer/cathode
(8) Anode/hole injecting layer/hole transporting layer/light emitting layer/electron injecting layer/cathode
(9) Anode/insulating layer/light emitting layer/insulating layer/cathode
(10) Anode/inorganic semiconductor layer/insulating layer/light emitting layer/insulating layer/cathode
(11) Anode/organic semiconductor layer/insulating layer/light emitting layer/insulating layer/cathode
(12) Anode/insulating layer/hole injecting layer/hole transporting layer/light emitting layer/insulating layer/cathode
(13) Anode/insulating layer/hole injecting layer/hole transporting layer/light emitting layer/electron injecting layer/cathode

Among them, usually the structure of (8) is preferably used, but it shall not be restricted to them.

The aromatic amine derivative of the present invention can be used in the light emitting zone or the hole transporting zone of the organic EL element, and it is used preferably in the hole transporting zone, particularly in the hole transporting layer, whereby capable of being obtained is the organic EL element which can emit blue light under a high temperature while maintaining a high luminous efficiency even at a low voltage and a long life.

An amount of the aromatic amine derivative of the present invention which is added to the light emitting zone or the hole transporting zone is preferably 30 to 100 mole %.

### (2) Light transmitting substrate

The organic EL element of the present invention is prepared on a light transmitting substrate. The light transmitting substrate referred to in this case is a substrate supporting the organic EL element, and it is preferably a flat substrate in which light in a visible region of 400 to 700 nm has a transmission factor of 50 % or more.

To be specific, it includes a glass plate, a polymer plate and the like. In particular, the glass plate includes soda lime glass, barium·strontium-containing glass, lead glass, aluminosilicate glass, borosilicate glass, barium borosilicate glass and quartz. The polymer plate includes polycarbonate, acryl, polyethylene terephthalate, polyether sulfide and polysulfone.

### (3) Anode

An anode in the organic EL element of the present invention has a function to inject a hole into the hole transporting layer or the light emitting layer, and it is effective that the anode has a work function of 4.5 eV or more. The specific examples of a material for the anode used in the present invention includes indium tin oxide alloy (ITO), zinc oxide (NESA), gold, silver, platinum and copper.

The anode can be prepared by forming a thin film of the above electrode substances by a method such as a deposition method or a sputtering method.

When light emitted from the light emitting layer is taken out from the anode, a transmission factor of the anode based on light emitted is preferably larger than 10 %. A sheet resistance of the anode is preferably several hundred Ω/□ or less. A film thickness of the anode is selected, though depending on the material, in a range of usually 10 nm to 1 µm, preferably 10 to 200 nm.

### (4) Light emitting layer

The light emitting layer in the organic EL element has the following functions of (1) to (3) in combination.
(1) Injecting function: a function in which a hole can be injected from an anode or a hole injecting layer in applying an electric field and in which an electron can be injected from a cathode or an electron injecting layer.
(2) Transporting function: a function in which a charge injected (electron and hole) can be migrated by virtue of a force of an electric field.
(3) Light emitting function: a function in which a field for recombination of an electron and a hole is provided and in which this is connected to light emission.

Provided that a difference between an easiness in injection of a hole and an easiness in injection of an electron may be present and that a difference may be present in a transporting ability shown by the mobilities of a hole and an electron, but any one of the charges is preferably migrated.

A publicly known method such as, for example, a deposition method, a spin coating method and an LB method can be applied as a method for forming the above light emitting layer. In particular, the light emitting layer is preferably a molecular volume film. In this case, the molecular volume film means a thin film formed by depositing a material compound staying in a gas phase state and a film formed by solidifying a material compound staying in a solution state or a liquid phase state, and the above molecular volume film can be distinguished from a thin film (molecular volume film) formed by the LB method by a difference in an aggregation structure and a higher order structure and a functional difference originating in it.

Further, as disclosed in Japanese Patent Application Laid-Open No. 51781/1982, the light emitting layer can be formed as well by dissolving a binding agent such as a resin and the material compound in a solvent to prepare a solution and then coating the solution by a spin coating method to form a thin film.

In the present invention, another publicly known light emitting materials other than the light emitting material comprising the aromatic amine derivative of the present invention may be added, if necessary, to the light emitting layer as long as the object of the present invention is not damaged. Further, a light emitting layer containing a different publicly known light emitting material may be laminated on the light emitting layer containing the light emitting material comprising the aromatic amine derivative of the present invention.

### (5) Hole injecting and transporting layer (hole transporting zone)

The hole injecting and transporting layers are layers for assisting injection of a hole into the light emitting layer to transport it to the light emitting region, and they have a large hole mobility and show a small ionization energy of usually 5.5 eV or less. A material which transports a hole to the light emitting layer by a lower electric field strength is preferred as the above hole injecting and transporting layers, and more preferred is a material in which a mobility of a hole is at least 10⁻⁴ cm²/V·second in applying an electric field of, for example, 10⁴ to 10⁶ V/cm.

When the aromatic amine derivative of the resent invention is used in the hole transporting zone, the hole injecting and transporting layers may be formed from the aromatic amine derivative of the resent invention alone or it may be used in a mixture with other materials.

The materials for forming the hole injecting and transporting layers by mixing with the aromatic amine derivative of the resent invention shall not specifically be restricted as long as they have the preferred properties described above, and capable of being used are optional materials selected from materials which have so far been used as charge transporting materials of holes in photoconductive materials and publicly known materials which are used for a hole injecting layer in an organic EL element.

The specific examples thereof include triazole derivatives (refer to U.S. Patent 3,112,197), oxadiazole derivatives (refer to U.S. Patent 3,189,447), imidazole derivatives (refer to Japanese Patent Publication No. 16096/1962), polyarylalkane derivatives (refer to U.S. Patent 3,615,402, ditto 3,820,989 and ditto 3,542,544, Japanese Patent Publication No. 555/1970 and ditto 10983/1976 and Japanese Patent Application Laid-Open No. 93224/1976, ditto 17105/1980, ditto 4148/1981, ditto 108667/1980, ditto 156953/1980 and ditto 36656/1981), pyrazoline derivatives and pyrazolone derivatives (refer to U.S. Patent 3,180,729 and ditto 4,278,746 and Japanese Patent Application Laid-Open No. 88064/1980, ditto 88065/1980, ditto 105537/1974, ditto 51086/1980, ditto 80051/1981, ditto 88141/1981, ditto 45545/1982, ditto 112637/1979 and ditto 74546/1980), phenylenediamine derivatives (refer to U.S. Patent 3,615,404, Japanese Patent Publication No. 10105/1976, ditto 3712/1971 and ditto 25336/1972 and Japanese Patent Application Laid-Open No. 53435/1979, ditto 110536/1979 and ditto 119925/1979), arylamine derivatives (refer to U.S. Patent 3,567,450, ditto 3,180,703, ditto 3,240,597, ditto 3,658,520, ditto 4,232,103, ditto 4,175,961 and ditto 4,012,376, Japanese Patent Publication No. 35702/1974 and ditto 27577/1964, Japanese Patent Application Laid-Open No. 144250/1980, ditto 119132/1981 and ditto 22437/1981 and German Patent 1,110,518), amino-substituted chalcone derivatives (refer to U.S. Patent 3,526,501), oxazole derivatives (disclosed in U.S. Patent 3,257,203), styrylanthracene derivatives (refer to Japanese Patent Application Laid-Open No. 46234/1981), fluorenone derivatives (refer to Japanese Patent Application Laid-Open No. 110837/1979), hydrazone derivatives (refer to U.S. Patent 3,717,462, Japanese Patent Application Laid-Open No. 59143/1979, ditto 52063/1980, ditto 52064/1980, ditto 46760/1980, ditto 85495/1980, ditto 11350/1982 and ditto 148749/1982 and Japanese Patent Application Laid-Open No. 311591/1990), stilbene derivatives (Japanese Patent Application Laid-Open No. 210363/1986, ditto 228451/1986, ditto 14642/1986, ditto 72255/1986, ditto 47646/1987, ditto 36674/1987, ditto 10652/1987, ditto 30255/1987, ditto 93455/1985, ditto 94462/1985, ditto 174749/1985 and ditto 175052/1985), silazane derivatives (refer to U.S. Patent 4,950,950), polysilane base (refer to Japanese Patent Application Laid-Open No. 204996/1990), aniline base copolymers (refer to Japanese Patent Application Laid-Open No. 282263/1990) and electroconductive high molecular oligomers (particularly thiophene oligomers) disclosed in Japanese Patent Application Laid-Open No. 211399/1989.

The compounds described above can be used as the material for the hole injecting layer, and preferably used are porphyrin compounds (disclosed in Japanese Patent Application Laid-Open No. 256965/1988), aromatic tertiary amine compounds and styrylamine compounds (refer to U.S. Patent 4,127,412 and Japanese Patent Application Laid-Open No. 27033/1978, ditto 58445/1979, ditto 149634/1979, ditto 64299/1979, ditto 79450/1980, ditto 144250/1980, ditto 119132/1981, ditto 295558/1986, ditto 98353/1986 and ditto 295695/1988), and the aromatic tertiary amine compounds are particularly preferably used.

Further, capable of being given are compounds having two condensed aromatic rings in a molecule described in U.S. Patent 5,061,569, for example, 4,4'-bis(N-(1-naphthyl)-N-phenylamino)biphenyl (hereinafter abbreviated as NPD) and 4,4',4"-tris(N-(3-methylphenyl)-N-phenylamino)triphenylamine (hereinafter abbreviated as MTDATA) in which three triphenylamine units are combined in the form of a star burst type disclosed in Japanese Patent Application Laid-Open No. 308688/1992.

Further, inorganic compounds such as p type Si, p type SiC and the like can also be used as the material for the hole injecting layer in addition to the aromatic dimethylidene base compounds described above as the material for the light emitting layer.

The hole injecting and transporting layers can be formed by making a thin film from the aromatic amine derivative of the resent invention by a publicly known method such as, for example, a vacuum deposition method, a spin coating method, a casting method and an LB method. A film thickness of the hole injecting and transporting layers shall not specifically be restricted, and it is usually 5 nm to 5 µm. The above hole injecting and transporting layers may be constituted from a single layer comprising at least one of the materials described above as long as the aromatic amine derivative of the resent invention is contained in the hole transporting zone, and hole injecting and transporting layers comprising compounds which are different from those used in the hole injecting and transporting layers described above may be laminated thereon.

Further, an organic semiconductor layer may be provided as a layer for assisting injection of a hole or injection of an electron into the light emitting layer, and the layer having a conductance of 10⁻¹⁰ S/cm or more is suited. Capable being used as the material for the above organic semiconductor layer are conductive oligomers such as thiophene-containing oligomers and arylamine-containing oligomers disclosed in Japanese Patent Application Laid-Open No. 193191/1996 and conductive dendrimers such as arylamine-containing dendrimers.

### (6) Electron injecting layer

The electron injecting and transporting layers are layers for assisting injection of an electron into the light emitting layer to transport it to the light emitting region, and they have a large electron mobility. Also, the adhesion improving layer is a layer comprising particularly a material having a good adhesive property with the cathode in the above electron injecting layer. The metal complexes of 8-hydroxyquinoline or the derivatives thereof are suited as a material used for the electron injecting layer.

The specific examples of the above metal complexes of 8-hydroxyquinoline or the derivatives thereof include metal chelate oxynoid compounds containing chelates of oxine (in general, 8-quinolinol or 8-hydroxyquinoline), and, for example, tris(8-quinolinol)aluminum (Alq) can be used as the electron injecting material.

The oxadiazole derivative includes an electron transmitting compound represented by the following formula: 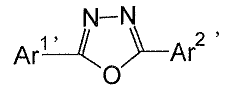 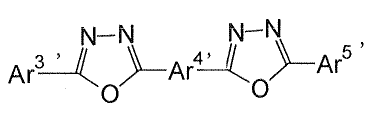 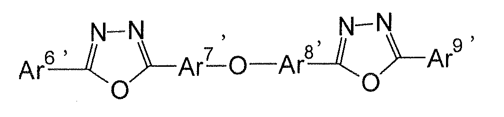 wherein Ar^{1'}, Ar^{2'}, Ar^{3'}, Ar^{5'}, Ar^{6'} and Ar^{9'} each represent a substituted or non-substituted aryl group, and they may be the same as or different from each other; Ar^{4'}, Ar^{7'} and Ar^{8'} each represent a substituted or non-substituted arylene group, and they may be the same as or different from each other.

In this connection, the aryl group includes phenyl, biphenyl, anthranyl, perylenyl and pyrenyl, and the arylene group includes phenylene, naphthylene, biphenylene, anthracenylene, perylenylene and pyrenylene. The substituents therefor include an alkyl group having 1 to 10 carbon atoms, an alkoxy group having 1 to 10 carbon atoms and a cyano group. The above electron transmitting compounds have preferably a thin film-forming property.

The following compounds can be given as the specific examples of the above electron transmitting compounds:

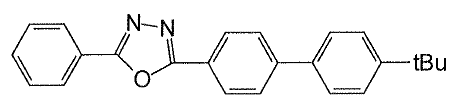

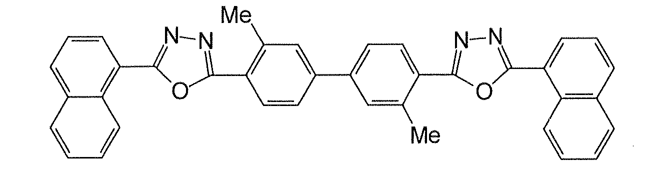

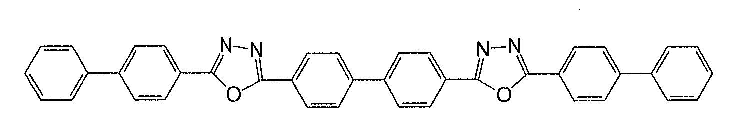

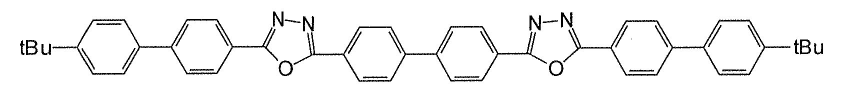

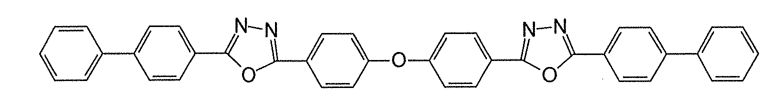

The organic EL element of the present invention may contain a reducing dopant in the region which transports an electron or an interfacial region between the cathode and the organic thin film layer. In this case, the reducing dopant is defined by a substance which can reduce an electron transporting compound. Accordingly, various compounds can be used as long as they have a reducing property of some extent, and capable of being suitably used is at least one substance selected from the group consisting of, for example, alkali metals, alkali earth metals, rare earth metals, oxides of alkali metals, halides of alkali metals, oxides of alkali earth metals, halides of alkali earth metals, oxides of rare earth metals or halides of rare earth metals, organic complexes of alkali metals, organic complexes of alkali earth metals and organic complexes of rare earth metals.

The specific examples of the preferred reducing dopant include at least one alkali metal selected from the group consisting of Na (work function: 2.36 eV), K (work function: 2.28 eV), Rb (work function: 2.16 eV) and Cs (work function: 1.95 eV) and at least one alkali earth metal selected from the group consisting of Ca (work function: 2.9 eV), Sr (work function: 2.0 to 2.5 eV) and Ba (work function: 2.52 eV), and the compounds having a work function of 2.9 eV or less are particularly preferred. Among them, the more preferred reducing dopant is at least one alkali metal selected from the group consisting of K, Rb and Cs, and it is more preferably Rb or Cs. It is most preferably Cs. The above alkali metals have a particularly high reducing ability, and addition of a relatively small amount thereof to the electron injecting zone makes it possible to raise a light emitting luminance and extend the life thereof in the organic EL element. The combination of two or more kinds of the above alkali metals is preferred as the reducing dopant having a work function of 2.9 eV or less, and particularly preferred is the combination containing Cs, for example, Cs and Na, Cs and K, Cs and Rb or Cs, Na and K. Containing Cs in combination makes it possible to efficiently exhibit the reducing ability, and addition thereof to the electron injecting zone makes it possible to raise a light emitting luminance and extend the life thereof in the organic EL element.

In the organic EL element of the present invention, an electron injecting layer constituted from an insulator and a semiconductor may further be provided between the cathode and the organic layer. This makes it possible to effectively prevent an electric current from leaking to raise the electron injecting property. Preferably used as the above insulator is at least one metal compound selected from the group consisting of alkali metal chalcogenides, alkali earth metal chalcogenides, halides of alkali metals and halides of alkali earth metals. If the electron injecting layer is constituted from the above alkali metal chalcogenides, the electron injecting property can further be enhanced, and therefore it is preferred. To be specific, the preferred alkali metal chalcogenides include, for example, Li₂O, LiO, Na₂S, Na₂Se and NaO, and the preferred alkali earth metal chalcogenides include, for example, CaO, BaO, SrO, BeO, BaS and CaSe. Also, the preferred halides of alkali metals include, for example, LiF, NaF, KF, LiCl, KCl and NaCl. The preferred halides of alkali earth metals include, for example, fluorides such as CaF₂, BaF₂, SrF₂, MgF₂ and BeF₂ and halides other than the fluorides.

The semiconductor constituting the electron transporting layer includes one of oxides, nitrides or nitride oxides containing at least one element of Ba, Ca, Sr, Yb, Al, Ga, In, Li, Na, Cd, Mg, Si, Ta, Sb and Zn or combinations of two or more kinds thereof. The inorganic compound constituting the electron transporting layer is preferably a microcrystalline or amorphous insulating thin film. If the electron transporting layer is constituted from the above insulating thin film, the more homogeneous thin film is formed, and therefore picture element defects such as dark spots can be reduced. The above inorganic compound includes the alkali metal chalcogenides, the alkali earth metal chalcogenides, the halides of alkali metals and the halides of alkali earth metals each described above.

### (7) Cathode

Substances using metals, alloys, electroconductive compounds and mixtures thereof each having a small work function (4 eV or less) for the electrode material are used as the cathode in order to inject electrons into the electron transporting layer or the light emitting layer. The specific examples of the above electrode material include sodium, sodium·potassium alloys, magnesium, lithium, magnesium·silver alloys, aluminum/aluminum oxide, aluminum·lithium alloys, indium and rare earth metals.

The above cathode can be prepared by forming a thin film from the above electrode materials by a method such as deposition and sputtering.

In this respect, when light emitted from the light emitting layer is taken out from the cathode, a transmission factor of the cathode based on light emitted is preferably larger than 10 %.

A sheet resistance of the cathode is preferably several hundred Ω/□ or less, and a film thickness thereof is usually 10 nm to 1 µm, preferably 50 to 200 nm.

### (8) Insulating layer

The organic EL element is liable to cause picture element defects by leak and short. In order to prevent this, an insulating thin film is preferably interposed between a pair of the electrodes.

A material used for the insulating layer includes, for example, aluminum oxide, lithium fluoride, lithium oxide, cesium fluoride, cesium oxide, magnesium oxide, magnesium fluoride, calcium oxide, calcium fluoride, aluminum nitride, titanium oxide, silicon oxide, germanium oxide, silicon nitride, boron nitride, molybdenum oxide, ruthenium oxide and vanadium oxide, and mixture s and laminates thereof may be used.

### (9) Production process for organic EL element

According to the materials and the forming methods which have been exemplified above, the anode, the light emitting layer, if necessary, the hole injecting and transporting layers and, if necessary, the electro injecting and transporting layers are formed, and further the cathode is formed, whereby the organic EL element can be prepared. Also, the organic EL element can be prepared from the cathode to the anode in an order which is reverse to what was described above.

A preparation example of an organic EL element having a structure in which an anode/a hole injecting layer/a light emitting layer/an electron injecting layer/a cathode are provided in order on a light transmitting substrate shall be described below.

First, a thin film comprising an anode material is formed on a suitable light transmitting substrate by a method such as deposition and sputtering so that a film thickness falls in a range of 1 µm or less, preferably 10 to 200 nm, whereby an anode is prepared. Next, a hole injecting layer is provided on this anode. The hole injecting layer can be formed, as described above, by a method such as a vacuum deposition method, a spin coating method, a casting method and an LB method, and it is preferably formed by the vacuum deposition method from the viewpoints that the homogeneous film is liable to be obtained and that pinholes are less liable to be produced. When forming the hole injecting layer by the vacuum deposition method, the depositing conditions thereof are varied according to the compounds used (materials for the hole injecting layer) and the crystal structure of the intended hole injecting layer, and in general, they are suitably selected preferably in the ranges of a depositing source temperature of 50 to 450°C, a vacuum degree of 10⁻⁷ to 10⁻³ torr, a depositing speed of 0.01 to 50 nm/second, a substrate temperature of -50 to 300°C and a film thickness of 5 nm to 5 µm.

Next, a light emitting layer can be formed by making a thin film from the desired organic light emitting material by a method such as a vacuum deposition method, a spin coating method and a casting method, whereby the light emitting layer is provided on the hole injecting layer, and it is preferably formed by the vacuum deposition method from the viewpoints that the homogeneous film is liable to be obtained and that pinholes are less liable to be produced. When forming the light emitting layer by the vacuum deposition method, the depositing conditions thereof are varied according to the compounds used, and in general, they can be selected from the same condition ranges as in the hole injecting layer.

Next, an electron injecting layer is provided on the above light emitting layer. It is preferably formed by the vacuum deposition method as is the case with the hole injecting layer and the light emitting layer since a homogeneous film has to be obtained. The depositing conditions thereof can be selected from the same condition ranges as in the hole injecting layer and the light emitting layer.

The aromatic amine derivative of the present invention can be codeposited together with the other materials, though varied depending on that it is added to any layer of the light emitting layer and the hole injecting layer, when using the vacuum deposition method. When using the spin coating method, it can be added by mixing with the other materials.

Lastly, a cathode is laminated, whereby an organic EL element can be obtained.

The cathode is constituted from metal, and therefore the deposition method and the sputtering method can be used. However, the vacuum deposition method is preferred in order to protect the organic substance layer of the base from being damaged in making the film.

The above organic EL element is preferably prepared serially from the anode up to the cathode in one vacuuming.

The forming methods of the respective layers in the organic EL element of the present invention shall not specifically be restricted, and the forming methods carried out by the vacuum deposition method and the spin coating method which have so far publicly been known can be used. The organic thin film containing the compound represented by Formula (1) described above which is used for the organic EL element of the present invention can be formed by a publicly known method carried out by a coating method such as a vacuum deposition method, a molecular beam evaporation method (MBE method), a dipping method using a solution prepared by dissolving the compound in a solvent, a spin coating method, a casting method, a bar coating method and a roll coating method.

The film thicknesses of the respective organic layers in the organic EL element of the present invention shall not specifically be restricted, and in general, if the film thickness is too small, defects such as pinholes are liable to be caused. On the other hand, if it is too large, high voltage has to be applied, and the efficiency is deteriorated, so that it falls preferably in a range of several nm to 1 µm.

When applying a direct voltage to the organic EL element, light emission can be observed by applying a voltage of 5 to 40 V setting a polarity of the anode to plus and that of the cathode to minus. An electric current does not flow by applying a voltage with the reverse polarities, and light emission is not caused at all. Further, when applying an AC voltage, uniform light emission can be observed only when the anode has a polarity of plus and the cathode has a polarity of minus. The waveform of an alternating current applied may be optional.

### EXAMPLES

Next, the present invention shall be explained in further details with reference to examples, but the present invention shall by no means be restricted by these examples.

### Synthetic Example 1 synthesis of 2-iodonaphthalene

A small amount of iodine (manufactured by Tokyo Kasei Kogyo Co., Ltd.) was added to 14 g of magnesium having a shaved form (manufactured by Tokyo Kasei Kogyo Co., Ltd.) and 230 ml of THF which was dried and distilled while heating at 50°C and stirring to activate magnesium, and then a solution prepared by dissolving 105 g of 2-bromonaphthalene (manufactured by Tokyo Kasei Kogyo Co., Ltd.) in one liter of THF which was dried and distilled was dropwise added thereto in one hour.

After finishing dropwise adding, the solution was stirred at 50°C for 2 hours and cooled down to - 10°C, and then 250 g of iodine was added little by little. The temperature was returned to a room temperature, and then stirring was continued for 2 hours.

Water 100 ml was added to the above reaction liquid, and it was extracted with ethyl acetate. The ethyl acetate layer was extracted with a caustic soda aqueous solution, and after the aqueous layer was washed with hexane, it was acidified with hydrochloric acid and then extracted with ethyl acetate. After the extract was concentrated under reduced pressure, the resulting viscous liquid was dissolved again in a caustic soda aqueous solution and precipitated with an acid to obtain 82 g of 2-iodonaphthalene.

### Synthetic Example 2 synthesis of 9-iodophenanthrene

A small amount of iodine (manufactured by Tokyo Kasei Kogyo Co., Ltd.) was added to 14 g of magnesium having a shaved form (manufactured by Tokyo Kasei Kogyo Co., Ltd.) and 230 ml of THF which was dried and distilled while heating at 50°C and stirring to activate magnesium, and then a solution prepared by dissolving 129 g of 9-bromophenanthrene (manufactured by Tokyo Kasei Kogyo Co., Ltd.) in one liter of THF which was dried and distilled was dropwise added thereto in one hour.

After finishing dropwise adding, the solution was stirred at 50°C for 2 hours and cooled down to - 10°C, and then 250 g of iodine was added little by little. The temperature was returned to a room temperature, and then stirring was continued for 2 hours.

Water 100 ml was added to the above reaction liquid, and it was extracted with ethyl acetate. The ethyl acetate layer was extracted with a caustic soda aqueous solution, and after the aqueous layer was washed with hexane, it was acidified with hydrochloric acid and then extracted with ethyl acetate. After the extract was concentrated under reduced pressure, the resulting viscous liquid was dissolved again in a caustic soda aqueous solution and precipitated with an acid to obtain 91 g of 9-iodophenanthrene.

### Synthetic Example 3 synthesis of N,N'-bis(naphtho-1-yl)-4,4'-benzidine (A1)

Mixed under argon flow were 100 g of N,N'-diacetyl-4,4'-benzidine (manufactured by Tokyo Kasei Kogyo Co., Ltd.), 282 g of 1-iodonaphthalene (manufactured by Tokyo Kasei Kogyo Co., Ltd.), 204 g of anhydrous potassium carbonate (manufactured by Tokyo Kasei Kogyo Co., Ltd.), 4.7 g of copper powder (manufactured by Hiroshima Wako Co., Ltd.) and 750 ml of decalin, and they were reacted at 190°C for 3 days.

After cooling, 2 liter of toluene was added thereto to filter an insoluble matter. The matter filtered was dissolved in 4.5 liter of chloroform to filter off an insoluble matter, and then the filtrate was treated with activated carbon and concentrated under reduced pressure. Acetone 3 litter was added when the solution became slurry in the middle of concentration, and crystal precipitated was filtered and dried.

This was suspended in a mixture of 2 liter of ethylene glycol and 20 ml of water, and 110 g of a 85 % potassium hydroxide aqueous solution was added thereto, followed by carrying out reaction at 120°C for 12 hours.

After left cooling down to a room temperature, the reaction liquid was injected to 4 liter of water and treated with activated carbon, and then it was concentrated under reduced pressure. Acetone was added when the solution became slurry, and crystal deposited was filtered and dried to obtain 108 g of N,N'-bis(naphtho-1-yl)-4,4'-benzidine (A1).

### Synthetic Example 4 synthesis of N,N'-bis(naphtho-2-yl)-4,4'-benzidine (A2)

Mixed under argon flow were 100 g of N,N'-diacetyl-4,4'-benzidine (manufactured by Tokyo Kasei Kogyo Co., Ltd.), 282 g of 2-iodonaphthalene, 204 g of anhydrous potassium carbonate (manufactured by Tokyo Kasei Kogyo Co., Ltd.), 4.7 g of copper powder (manufactured by Hiroshima Wako Co., Ltd.) and 750 ml of decalin, and they were reacted at 190°C for 3 days.

After cooling, 2 liter of toluene was added thereto to filter an insoluble matter. The matter filtered was dissolved in 4.5 liter of chloroform to filter off an insoluble matter, and then the filtrate was treated with activated carbon and concentrated under reduced pressure. Acetone 3 litter was added when the solution became slurry in the middle of concentration, and crystal deposited was filtered and dried.

This was suspended in a mixture of 2 liter of ethylene glycol and 20 ml of water, and 110 g of a 85 % potassium hydroxide aqueous solution was added thereto, followed by carrying out reaction at 120°C for 12 hours.

After left cooling down to a room temperature, the reaction liquid was injected to 4 liter of water and treated with activated carbon, and then it was concentrated under reduced pressure. Acetone was added when the solution became slurry, and crystal deposited was filtered and dried to obtain 104 g of N,N'-bis(naphtho-2-yl)-4,4'-benzidine (A2).

### Synthetic Example 5 synthesis of N,N'-bis(phenantho-9-yl)-4,4'-benzidine (A3)

Mixed under argon flow were 100 g of N,N'-diacetyl-4,4'-benzidine (manufactured by Tokyo Kasei Kogyo Co., Ltd.), 337 g of 9-iodophenanthrene, 204 g of anhydrous potassium carbonate (manufactured by Tokyo Kasei Kogyo Co., Ltd.), 4.7 g of copper powder (manufactured by Hiroshima Wako Co., Ltd.) and 750 ml of decalin, and they were reacted at 190°C for 3 days.

After cooling, 2 liter of toluene was added thereto to filter an insoluble matter. The matter filtered was dissolved in 4.5 liter of chloroform to filter off an insoluble matter, and then the filtrate was treated with activated carbon and concentrated under reduced pressure. Acetone 3 litter was added when the solution became slurry in the middle of concentration, and crystal deposited was filtered and dried.

This was suspended in a mixture of 2 liter of ethylene glycol and 20 ml of water, and 110 g of a 85 % potassium hydroxide aqueous solution was added thereto, followed by carrying out reaction at 120°C for 12 hours.

After left cooling down to a room temperature, the reaction liquid was injected to 4 liter of water and treated with activated carbon, and then it was concentrated under reduced pressure. Acetone was added when the solution became slurry, and crystal deposited was filtered and dried to obtain 116 g of N,N'-bis(phenantho-9-yl)-4,4'-benzidine (A3).

### Synthetic Example 6 synthesis of 4-bromodiphenylamine (A4)

Mixed under argon flow were 100 g of acetanilide (manufactured by Hiroshima Wako Co., Ltd.), 314 g of 4-bromoiodobenzene, 110 g of anhydrous potassium carbonate (manufactured by Tokyo Kasei Kogyo Co., Ltd.), 4.7 g of copper powder (manufactured by Hiroshima Wako Co., Ltd.) and 750 ml of decalin, and they were reacted at 190°C for 3 days.

After cooling, 2 liter of toluene was added thereto to filter an insoluble matter. The matter filtered was dissolved in 4.5 liter of chloroform to filter off an insoluble matter, and then the filtrate was treated with activated carbon and concentrated under reduced pressure. Acetone 3 litter was added when the solution became slurry in the middle of concentration, and crystal deposited was filtered and dried.

This was suspended in a mixture of 2 liter of ethylene glycol and 20 ml of water, and 110 g of a 85 % potassium hydroxide aqueous solution was added thereto, followed by carrying out reaction at 120°C for 12 hours.

After left cooling down to a room temperature, the reaction liquid was injected to 4 liter of water and treated with activated carbon, and then it was concentrated under reduced pressure. Acetone was added when the solution became slurry, and crystal deposited was filtered and dried to obtain 96 g of 4-bromodiphenylamine (A4).

### Synthetic Example 7 synthesis of N-(4-bromophenyl)-N-phenyl-1-naphthylamine (B1)

Mixed under argon flow were 259 g of N-phenyl-1-naphthylamine (manufactured by Tokyo Kasei Kogyo Co., Ltd.), 279 g of 4-dibromobenzene (manufactured by Tokyo Kasei Kogyo Co., Ltd.), 136 g of sodium t-butoxide (manufactured by Tokyo Kasei Kogyo Co., Ltd.), 17 g of bis(triphenylphosphine)palladium dichloride (manufactured by Tokyo Kasei Kogyo Co., Ltd.) and 4.6 liter of xylene, and they were reacted at 130°C for 12 hours.

Water was added to the reaction liquid, and the mixture was filtered through celite, followed by carrying out separation thereof with toluene. The oil layer was concentrated under reduced pressure to obtain crystal. This was refined through a column and then dissolved in toluene, and hexane was added thereto to reprecipitate crystal. It was filtered and then dried to obtain 135 g of N-(4-bromophenyl)-N-phenyl-1-naphthylamine (B1).

### Synthetic Example 8 synthesis of N-(4-bromophenyl)-N-phenyl-2-naphthylamine (B2)

Mixed under argon flow were 259 g of N-phenyl-2-naphthylamine (manufactured by Tokyo Kasei Kogyo Co., Ltd.), 279 g of 1,4-dibromobenzene (manufactured by Tokyo Kasei Kogyo Co., Ltd.), 136 g of sodium t-butoxide (manufactured by Tokyo Kasei Kogyo Co., Ltd.), 17 g of bis(triphenylphosphine)palladium dichloride (manufactured by Tokyo Kasei Kogyo Co., Ltd.) and 4.6 liter of xylene, and they were reacted at 130°C for 12 hours.

Water was added to the reaction liquid, and the mixture was filtered through celite, followed by carrying out separation thereof with toluene. The oil layer was concentrated under reduced pressure to obtain crystal. This was refined through a column and then dissolved in toluene, and hexane was added thereto to reprecipitate crystal. It was filtered and then dried to obtain 145 g of N-(4-bromophenyl)-N-phenyl-2-naphthylamine (B2).

### Synthetic Example 9 synthesis of N-(4-bromophenyl)-N-phenyl-9-aminophenanthrene (B3)

Mixed under argon flow were 73 g of 4-bromodiphenylamine (A4), 135 g of 9-iodophenanthrene, 34 g of sodium t-butoxide (manufactured by Tokyo Kasei Kogyo Co., Ltd.), 4 g of bis(triphenylphosphine)palladium dichloride (manufactured by Tokyo Kasei Kogyo Co., Ltd.) and 1.5 liter of xylene, and they were reacted at 130°C for 12 hours.

Water was added to the reaction liquid, and the mixture was filtered through celite, followed by carrying out separation thereof with toluene. The oil layer was concentrated under reduced pressure to obtain crystal. This was refined through a column and then dissolved in toluene, and hexane was added thereto to reprecipitate crystal. It was filtered and then dried to obtain 82 g of N-(4-bromophenyl)-N-phenyl-9-aminophenanthrene (B3).

### Synthetic Example 10 synthesis of N,N-di(naphtho-1-yl)-4-bromoaniline (B4)

Mixed under argon flow were 50 g of 4-bromoaniline (manufactured by Tokyo Kasei Kogyo Co., Ltd.), 221 g of 1-iodonaphthalene (manufactured by Tokyo Kasei Kogyo Co., Ltd.), 84 g of sodium t-butoxide (manufactured by Tokyo Kasei Kogyo Co., Ltd.), 11 g of bis(triphenylphosphine)palladium dichloride (manufactured by Tokyo Kasei Kogyo Co., Ltd.) and 4.5 liter of xylene, and they were reacted at 130°C for 12 hours.

Water was added to the reaction liquid, and the mixture was filtered through celite, followed by carrying out separation thereof with toluene. The oil layer was concentrated under reduced pressure to obtain crystal. This was refined through a column and then dissolved in toluene, and hexane was added thereto to reprecipitate crystal. It was filtered and then dried to obtain 90 g of N,N-di(naphtho-1-yl)-4-bromoaniline (B4).

### Synthetic Example 11 synthesis of N,N-di(naphtho-2-yl)-4-bromoaniline (B5)

Mixed under argon flow were 50 g of 4-bromoaniline (manufactured by Tokyo Kasei Kogyo Co., Ltd.), 221 g of 2-iodonaphthalene, 84 g of sodium t-butoxide (manufactured by Tokyo Kasei Kogyo Co., Ltd.), 11 g of bis(triphenylphosphine)palladium dichloride (manufactured by Tokyo Kasei Kogyo Co., Ltd.) and 4.5 liter of xylene, and they were reacted at 130°C for 12 hours.

Water was added to the reaction liquid, and the mixture was filtered through celite, followed by carrying out separation thereof with toluene. The oil layer was concentrated under reduced pressure to obtain crystal. This was refined through a column and then dissolved in toluene, and hexane was added thereto to reprecipitate crystal. It was filtered and then dried to obtain 84 g of N,N-di(naphtho-2-yl)-4-bromoaniline (B5).

### Synthetic Example 12 synthesis of N,N-di(phenantho-9-yl)-4-bromoaniline (B6)

Mixed under argon flow were 50 g of 4-bromoaniline (manufactured by Tokyo Kasei Kogyo Co., Ltd.), 265 g of 9-iodophenanthrene, 84 g of sodium t-butoxide (manufactured by Tokyo Kasei Kogyo Co., Ltd.), 11 g of bis(triphenylphosphine)palladium dichloride (manufactured by Tokyo Kasei Kogyo Co., Ltd.) and 4.5 liter of xylene, and they were reacted at 130°C for 12 hours.

Water was added to the reaction liquid, and the mixture was filtered through celite, followed by carrying out separation thereof with toluene. The oil layer was concentrated under reduced pressure to obtain crystal. This was refined through a column and then dissolved in toluene, and hexane was added thereto to reprecipitate crystal. It was filtered and then dried to obtain 101 g of N,N-di(phenantho-9-yl)-4-bromoaniline (B6).

### Synthetic Example 13 synthesis of N-(4-bromophenyl)-N-(naphtho-2-yl)-1-naphthylamine (B7)

Mixed under argon flow were 100 g of 2-aminonaphthalene (manufactured by Aldrich Co., Ltd.), 210 g of 1-iodonaphthalene (manufactured by Tokyo Kasei Kogyo Co., Ltd.), 204 g of anhydrous potassium carbonate (manufactured by Tokyo Kasei Kogyo Co., Ltd.), 4.7 g of copper powder (manufactured by Hiroshima Wako Co., Ltd.) and 750 ml of decalin, and they were reacted at 190°C for 3 days.

After cooling, 2 liter of toluene was added thereto to filter an insoluble matter. The matter filtered was dissolved in 4.5 liter of chloroform to filter off an insoluble matter, and then the filtrate was treated with activated carbon and concentrated under reduced pressure. Acetone 3 litter was added when the solution became slurry in the middle of concentration, and crystal deposited was filtered and refined through a column.

Mixed under argon flow were the powder obtained, 200 g of 4-bromoiodobenzene (manufactured by Tokyo Kasei Kogyo Co., Ltd.), 204 g of anhydrous potassium carbonate (manufactured by Tokyo Kasei Kogyo Co., Ltd.), 4.7 g of copper powder (manufactured by Hiroshima Wako Co., Ltd.) and 750 ml of decalin, and they were reacted at 190°C for 3 days.

After cooling, 2 liter of toluene was added thereto to filter an insoluble matter. The matter filtered was dissolved in 4.5 liter of chloroform to filter off an insoluble matter, and then the filtrate was treated with activated carbon and concentrated under reduced pressure. Acetone 3 litter was added when the solution became slurry in the middle of concentration, and crystal deposited was filtered and refined through a column. This was dissolved in toluene, and hexane was added thereto to reprecipitate crystal. It was filtered and then dried to obtain 68 g of N-(4-bromophenyl)-N-(naphtho-2-yl)-1-naphthylamine (B7).

### Synthetic Example 14 synthesis of N-(4-bromophenyl)-N-(phenantho-9-yl)-1-naphthylamine (B8)

Mixed under argon flow were 100 g of 1-aminonaphthalene (manufactured by Aldrich Co., Ltd.), 255 g of 9-iodophenanthrene, 204 g of anhydrous potassium carbonate (manufactured by Tokyo Kasei Kogyo Co., Ltd.), 4.7 g of copper powder (manufactured by Hiroshima Wako Co., Ltd.) and 750 ml of decalin, and they were reacted at 190°C for 3 days.

After cooling, 2 liter of toluene was added thereto to filter an insoluble matter. The matter filtered was dissolved in 4.5 liter of chloroform to filter off an insoluble matter, and then the filtrate was treated with activated carbon and concentrated under reduced pressure. Acetone 3 litter was added when the solution became slurry in the middle of concentration, and crystal deposited was filtered and refined through a column.

Mixed under argon flow were the powder obtained, 200 g of 4-bromoiodobenzene (manufactured by Tokyo Kasei Kogyo Co., Ltd.), 204 g of anhydrous potassium carbonate (manufactured by Tokyo Kasei Kogyo Co., Ltd.), 4.7 g of copper powder (manufactured by Hiroshima Wako Co., Ltd.) and 750 ml of decalin, and they were reacted at 190°C for 3 days.

After cooling, 2 liter of toluene was added thereto to filter an insoluble matter. The matter filtered was dissolved in 4.5 liter of chloroform to filter off an insoluble matter, and then the filtrate was treated with activated carbon and concentrated under reduced pressure. Acetone 3 litter was added when the solution became slurry in the middle of concentration, and crystal deposited was filtered. This was refined through a column and dissolved in toluene, and hexane was added thereto to reprecipitate crystal. It was filtered and then dried to obtain 64 g of N-(4-bromophenyl)-N-(phenantho-9-yl)-1-naphthylamine (B8).

### Synthetic Example 15 synthesis of N-(4-bromophenyl)-N-(phenantho-9-yl)-2-naphthylamine (B9)

Mixed under argon flow were 100 g of 2-aminonaphthalene (manufactured by Aldrich Co., Ltd.), 255 g of 9-iodophenanthrene, 204 g of anhydrous potassium carbonate (manufactured by Tokyo Kasei Kogyo Co., Ltd.), 4.7 g of copper powder (manufactured by Hiroshima Wako Co., Ltd.) and 750 ml of decalin, and they were reacted at 190°C for 3 days.

After cooling, 2 liter of toluene was added thereto to filter an insoluble matter. The matter filtered was dissolved in 4.5 liter of chloroform to filter off an insoluble matter, and then the filtrate was treated with activated carbon and concentrated under reduced pressure. Acetone 3 litter was added when the solution became slurry in the middle of concentration, and crystal deposited was filtered and refined through a column.

Mixed under argon flow were the powder obtained, 200 g of 4-bromoiodobenzene (manufactured by Tokyo Kasei Kogyo Co., Ltd.), 204 g of anhydrous potassium carbonate (manufactured by Tokyo Kasei Kogyo Co., Ltd.), 4.7 g of copper powder (manufactured by Hiroshima Wako Co., Ltd.) and 750 ml of decalin, and they were reacted at 190°C for 3 days.

After cooling, 2 liter of toluene was added thereto to filter an insoluble matter. The matter filtered was dissolved in 4.5 liter of chloroform to filter off an insoluble matter, and then the filtrate was treated with activated carbon and concentrated under reduced pressure. Acetone 3 litter was added when the solution became slurry in the middle of concentration, and crystal deposited was filtered. This was refined through a column and dissolved in toluene, and hexane was added thereto to reprecipitate crystal. It was filtered and then dried to obtain 66 g of N-(4-bromophenyl)-N-(phenantho-9-yl)-2-naphthylamine (B9).

### Synthetic Example 16 synthesis of 4-bromotriphenylamine (B10)

A solution of 15 g of bromine (manufactured by Hiroshima Wako Co., Ltd.) and 100 ml of chloroform was dropwise added to a chloroform solution of 20 g of triphenylamine (manufactured by Tokyo Kasei Kogyo Co., Ltd.) at 0°C in 15 minutes. Then, the solution was stirred at a room temperature for 30 minutes.

The reaction liquid was washed in order with 500 ml of water, 500 ml of a saturated sodium bicarbonate aqueous solution, 500 ml of a sodium thiosulfate aqueous solution and 500 ml of water, and it was dried on anhydrous magnesium sulfate and then concentrated under educed pressure. A crude product was recrystallized to obtain 19 g of 4-bromotriphenylamine (B10).

### Example 1 synthesis of compound (H1)

Mixed under argon flow were 10 g of the compound (A1), 21 g of the compound (B1), 6 g of sodium t-butoxide (manufactured by Hiroshima Wako Co., Ltd.), 1 g of bis(triphenylphosphine)palladium (II) chloride (manufactured by Tokyo Kasei Kogyo Co., Ltd.) and 500 ml of xylene, and they were reacted at 130°C for 24 hours.

After cooling, 1000 ml of water was added thereto, and the mixture was filtered through celite. The filtrate was extracted with toluene, and the extract was dried on anhydrous magnesium sulfate. This was concentrated under reduced pressure, and the resulting crude product was refined through a column and recrystallized from toluene. It was filtered and then dried to obtain 16 g of a pale yellow powder.

FD-MS (field desorption mass spectrum) of the powder thus obtained was measured to obtain a peak of m/z = 1023 versus C₇₆H₅₄N₄ = 1022, and therefore this was identified as the intended compound (H1).

### Example 2 synthesis of compound (H2)

Mixed under argon flow were 10 g of the compound (A1), 24 g of the compound (B4), 6 g of sodium t-butoxide (manufactured by Hiroshima Wako Co., Ltd.), 1 g of bis(triphenylphosphine)palladium (II) chloride (manufactured by Tokyo Kasei Kogyo Co., Ltd.) and 500 ml of xylene, and they were reacted at 130°C for 24 hours.

After cooling, 1000 ml of water was added thereto, and the mixture was filtered through celite. The filtrate was extracted with toluene, and the extract was dried on anhydrous magnesium sulfate. This was concentrated under reduced pressure, and the resulting crude product was refined through a column and recrystallized from toluene. It was filtered and then dried to obtain 18 g of a pale yellow powder.

FD-MS of the powder thus obtained was measured to obtain a peak of m/z = 1123 versus C₈₄H₅₈N₄ = 1122, and therefore this was identified as the intended compound (H2).

### Example 3 synthesis of compound (H3)

Mixed under argon flow were 10 g of the compound (A1), 21 g of the compound (B2), 6 g of sodium t-butoxide (manufactured by Hiroshima Wako Co., Ltd.), 1 g of bis(triphenylphosphine)palladium (II) chloride (manufactured by Tokyo Kasei Kogyo Co., Ltd.) and 500 ml of xylene, and they were reacted at 130°C for 24 hours.

After cooling, 1000 ml of water was added thereto, and the mixture was filtered through celite. The filtrate was extracted with toluene, and the extract was dried on anhydrous magnesium sulfate. This was concentrated under reduced pressure, and the resulting crude product was refined through a column and recrystallized from toluene. It was filtered and then dried to obtain 18 g of a pale yellow powder.

FD-MS of the powder thus obtained was measured to obtain a peak of m/z = 1023 versus C₇₈H₅₄N₄ = 1022, and therefore this was identified as the intended compound (H3).

### Example 4 synthesis of compound (H4)

Mixed under argon flow were 10 g of the compound (A1), 24 g of the compound (B7), 6 g of sodium t-butoxide (manufactured by Hiroshima Wako Co., Ltd.), 1 g of bis(triphenylphosphine)palladium (II) chloride (manufactured by Tokyo Kasei Kogyo Co., Ltd.) and 500 ml of xylene, and they were reacted at 130°C for 24 hours.

After cooling, 1000 ml of water was added thereto, and the mixture was filtered through celite. The filtrate was extracted with toluene, and the extract was dried on anhydrous magnesium sulfate. This was concentrated under reduced pressure, and the resulting crude product was refined through a column and recrystallized from toluene. It was filtered and then dried to obtain 18 g of a pale yellow powder.

FD-MS of the powder thus obtained was measured to obtain a peak of m/z = 1123 versus C₈₄H₅₈N₄ = 1122, and therefore this was identified as the intended compound (H4).

### Example 5 synthesis of compound (H5)

Mixed under argon flow were 10 g of the compound (A1), 24 g of the compound (B5), 6 g of sodium t-butoxide (manufactured by Hiroshima Wako Co., Ltd.), 1 g of bis(triphenylphosphine)palladium (II) chloride (manufactured by Tokyo Kasei Kogyo Co., Ltd.) and 500 ml of xylene, and they were reacted at 130°C for 24 hours.

After cooling, 1000 ml of water was added thereto, and the mixture was filtered through celite. The filtrate was extracted with toluene, and the extract was dried on anhydrous magnesium sulfate. This was concentrated under reduced pressure, and the resulting crude product was refined through a column and recrystallized from toluene. It was filtered and then dried to obtain 15 g of a pale yellow powder.

FD-MS of the powder thus obtained was measured to obtain a peak of m/z = 1123 versus C₈₄H₅₈N₄ = 1122, and therefore this was identified as the intended compound (H5).

### Example 6 synthesis of compound (H6)

Mixed under argon flow were 10 g of the compound (A1), 24 g of the compound (B3), 6 g of sodium t-butoxide (manufactured by Hiroshima Wako Co., Ltd.), 1 g of bis(triphenylphosphine)palladium (II) chloride (manufactured by Tokyo Kasei Kogyo Co., Ltd.) and 500 ml of xylene, and they were reacted at 130°C for 24 hours.

After cooling, 1000 ml of water was added thereto, and the mixture was filtered through celite. The filtrate was extracted with toluene, and the extract was dried on anhydrous magnesium sulfate. This was concentrated under reduced pressure, and the resulting crude product was refined through a column and recrystallized from toluene. It was filtered and then dried to obtain 16 g of a pale yellow powder.

FD-MS of the powder thus obtained was measured to obtain a peak of m/z = 1123 versus C₈₄H₅₈N₄ = 1122, and therefore this was identified as the intended compound (H6).

### Example 7 synthesis of compound (H7)

Mixed under argon flow were 10 g of the compound (A1), 27 g of the compound (B8), 6 g of sodium t-butoxide (manufactured by Hiroshima Wako Co., Ltd.), 1 g of bis(triphenylphosphine)palladium (II) chloride (manufactured by Tokyo Kasei Kogyo Co., Ltd.) and 500 ml of xylene, and they were reacted at 130°C for 24 hours.

After cooling, 1000 ml of water was added thereto, and the mixture was filtered through celite. The filtrate was extracted with toluene, and the extract was dried on anhydrous magnesium sulfate. This was concentrated under reduced pressure, and the resulting crude product was refined through a column and recrystallized from toluene. It was filtered and then dried to obtain 17 g of a pale yellow powder.

FD-MS of the powder thus obtained was measured to obtain a peak of m/z = 1223 versus C₉₂H₆₂N₄ = 1222, and therefore this was identified as the intended compound (H7).

### Example 8 synthesis of compound (H8)

Mixed under argon flow were 10 g of the compound (A1), 27 g of the compound (B9), 6 g of sodium t-butoxide (manufactured by Hiroshima Wako Co., Ltd.), 1 g of bis(triphenylphosphine)palladium (II) chloride (manufactured by Tokyo Kasei Kogyo Co., Ltd.) and 500 ml of xylene, and they were reacted at 130°C for 24 hours.

After cooling, 1000 ml of water was added thereto, and the mixture was filtered through celite. The filtrate was extracted with toluene, and the extract was dried on anhydrous magnesium sulfate. This was concentrated under reduced pressure, and the resulting crude product was refined through a column and recrystallized from toluene. It was filtered and then dried to obtain 19 g of a pale yellow powder.

FD-MS of the powder thus obtained was measured to obtain a peak of m/z = 1223 versus C₉₂H₆₂N₄ = 1222, and therefore this was identified as the intended compound (H8).

### Example 9 synthesis of compound (H9)

Mixed under argon flow were 10 g of the compound (A1), 30 g of the compound (B6), 6 g of sodium t-butoxide (manufactured by Hiroshima Wako Co., Ltd.), 1 g of bis(triphenylphosphine)palladium (II) chloride (manufactured by Tokyo Kasei Kogyo Co., Ltd.) and 500 ml of xylene, and they were reacted at 130°C for 24 hours.

After cooling, 1000 ml of water was added thereto, and the mixture was filtered through celite. The filtrate was extracted with toluene, and the extract was dried on anhydrous magnesium sulfate. This was concentrated under reduced pressure, and the resulting crude product was refined through a column and recrystallized from toluene. It was filtered and then dried to obtain 14 g of a pale yellow powder.

FD-MS of the powder thus obtained was measured to obtain a peak of m/z = 1323 versus C₁₀₀H₆₆N₄ = 1322, and therefore this was identified as the intended compound (H9).

### Example 10 synthesis of compound (H10)

Mixed under argon flow were 10 g of the compound (A2), 21 g of the compound (B1), 6 g of sodium t-butoxide (manufactured by Hiroshima Wako Co., Ltd.), 1 g of bis(triphenylphosphine)palladium (II) chloride (manufactured by Tokyo Kasei Kogyo Co., Ltd.) and 500 ml of xylene, and they were reacted at 130°C for 24 hours.

After cooling, 1000 ml of water was added thereto, and the mixture was filtered through celite. The filtrate was extracted with toluene, and the extract was dried on anhydrous magnesium sulfate. This was concentrated under reduced pressure, and the resulting crude product was refined through a column and recrystallized from toluene. It was filtered and then dried to obtain 20 g of a pale yellow powder.

FD-MS of the powder thus obtained was measured to obtain a peak of m/z = 1023 versus C₇₆H₅₄N₄ = 1022, and therefore this was identified as the intended compound (H10).

### Example 11 synthesis of compound (H11)

Mixed under argon flow were 10 g of the compound (A2), 24 g of the compound (B4), 6 g of sodium t-butoxide (manufactured by Hiroshima Wako Co., Ltd.), 1 g of bis(triphenylphosphine)palladium (II) chloride (manufactured by Tokyo Kasei Kogyo Co., Ltd.) and 500 ml of xylene, and they were reacted at 130°C for 24 hours.

After cooling, 1000 ml of water was added thereto, and the mixture was filtered through celite. The filtrate was extracted with toluene, and the extract was dried on anhydrous magnesium sulfate. This was concentrated under reduced pressure, and the resulting crude product was refined through a column and recrystallized from toluene. It was filtered and then dried to obtain 17 g of a pale yellow powder.

FD-MS of the powder thus obtained was measured to obtain a peak of m/z = 1123 versus C₈₄H₅₈N₄ = 1122, and therefore this was identified as the intended compound (H11).

### Example 12 synthesis of compound (H12)

Mixed under argon flow were 10 g of the compound (A2), 21 g of the compound (B2), 6 g of sodium t-butoxide (manufactured by Hiroshima Wako Co., Ltd.), 1 g of bis(triphenylphosphine)palladium (II) chloride (manufactured by Tokyo Kasei Kogyo Co., Ltd.) and 500 ml of xylene, and they were reacted at 130°C for 24 hours.

After cooling, 1000 ml of water was added thereto, and the mixture was filtered through celite. The filtrate was extracted with toluene, and the extract was dried on anhydrous magnesium sulfate. This was concentrated under reduced pressure, and the resulting crude product was refined through a column and recrystallized from toluene. It was filtered and then dried to obtain 19 g of a pale yellow powder.

FD-MS of the powder thus obtained was measured to obtain a peak of m/z = 1023 versus C₇₈H₅₄N₄ = 1022, and therefore this was identified as the intended compound (H12).

### Example 13 synthesis of compound (H13)

Mixed under argon flow were 10 g of the compound (A2), 24 g of the compound (B7), 6 g of sodium t-butoxide (manufactured by Hiroshima Wako Co., Ltd.), 1 g of bis(triphenylphosphine)palladium (II) chloride (manufactured by Tokyo Kasei Kogyo Co., Ltd.) and 500 ml of xylene, and they were reacted at 130°C for 24 hours.

After cooling, 1000 ml of water was added thereto, and the mixture was filtered through celite. The filtrate was extracted with toluene, and the extract was dried on anhydrous magnesium sulfate. This was concentrated under reduced pressure, and the resulting crude product was refined through a column and recrystallized from toluene. It was filtered and then dried to obtain 21 g of a pale yellow powder.

FD-MS of the powder thus obtained was measured to obtain a peak of m/z = 1123 versus C₈₄H₅₈N₄ = 1122, and therefore this was identified as the intended compound (H13).

### Example 14 synthesis of compound (H14)

Mixed under argon flow were 10 g of the compound (A2), 24 g of the compound (B5), 6 g of sodium t-butoxide (manufactured by Hiroshima Wako Co., Ltd.), 1 g of bis(triphenylphosphine)palladium (II) chloride (manufactured by Tokyo Kasei Kogyo Co., Ltd.) and 500 ml of xylene, and they were reacted at 130°C for 24 hours.

After cooling, 1000 ml of water was added thereto, and the mixture was filtered through celite. The filtrate was extracted with toluene, and the extract was dried on anhydrous magnesium sulfate. This was concentrated under reduced pressure, and the resulting crude product was refined through a column and recrystallized from toluene. It was filtered and then dried to obtain 18 g of a pale yellow powder.

FD-MS of the powder thus obtained was measured to obtain a peak of m/z = 1123 versus C₈₄H₅₈N₄ = 1122, and therefore this was identified as the intended compound (H14).

### Example 15 synthesis of compound (H15)

Mixed under argon flow were 10 g of the compound (A2), 24 g of the compound (B3), 6 g of sodium t-butoxide (manufactured by Hiroshima Wako Co., Ltd.), 1 g of bis(triphenylphosphine)palladium (II) chloride (manufactured by Tokyo Kasei Kogyo Co., Ltd.) and 500 ml of xylene, and they were reacted at 130°C for 24 hours.

After cooling, 1000 ml of water was added thereto, and the mixture was filtered through celite. The filtrate was extracted with toluene, and the extract was dried on anhydrous magnesium sulfate. This was concentrated under reduced pressure, and the resulting crude product was refined through a column and recrystallized from toluene. It was filtered and then dried to obtain 17 g of a pale yellow powder.

FD-MS of the powder thus obtained was measured to obtain a peak of m/z = 1123 versus C₈₄H₅₈N₄ = 1122, and therefore this was identified as the intended compound (H15).

### Example 16 synthesis of compound (H16)

Mixed under argon flow were 10 g of the compound (A2), 27 g of the compound (B8), 6 g of sodium t-butoxide (manufactured by Hiroshima Wako Co., Ltd.), 1 g of bis(triphenylphosphine)palladium (II) chloride (manufactured by Tokyo Kasei Kogyo Co., Ltd.) and 500 ml of xylene, and they were reacted at 130°C for 24 hours.

After cooling, 1000 ml of water was added thereto, and the mixture was filtered through celite. The filtrate was extracted with toluene, and the extract was dried on anhydrous magnesium sulfate. This was concentrated under reduced pressure, and the resulting crude product was refined through a column and recrystallized from toluene. It was filtered and then dried to obtain 17 g of a pale yellow powder.

FD-MS of the powder thus obtained was measured to obtain a peak of m/z = 1223 versus C₉₂H₆₂N₄ = 1222, and therefore this was identified as the intended compound (H16).

### Example 17 synthesis of compound (H17)

Mixed under argon flow were 10 g of the compound (A2), 27 g of the compound (B9), 6 g of sodium t-butoxide (manufactured by Hiroshima Wako Co., Ltd.), 1 g of bis(triphenylphosphine)palladium (II) chloride (manufactured by Tokyo Kasei Kogyo Co., Ltd.) and 500 ml of xylene, and they were reacted at 130°C for 24 hours.

After cooling, 1000 ml of water was added thereto, and the mixture was filtered through celite. The filtrate was extracted with toluene, and the extract was dried on anhydrous magnesium sulfate. This was concentrated under reduced pressure, and the resulting crude product was refined through a column and recrystallized from toluene. It was filtered and then dried to obtain 16 g of a pale yellow powder.

FD-MS of the powder thus obtained was measured to obtain a peak of m/z = 1223 versus C₉₂H₆₂N₄ = 1222, and therefore this was identified as the intended compound (H17).

### Example 18 synthesis of compound (H18)

Mixed under argon flow were 10 g of the compound (A2), 30 g of the compound (B6), 6 g of sodium t-butoxide (manufactured by Hiroshima Wako Co., Ltd.), 1 g of bis(triphenylphosphine)palladium (II) chloride (manufactured by Tokyo Kasei Kogyo Co., Ltd.) and 500 ml of xylene, and they were reacted at 130°C for 24 hours.

After cooling, 1000 ml of water was added thereto, and the mixture was filtered through celite. The filtrate was extracted with toluene, and the extract was dried on anhydrous magnesium sulfate. This was concentrated under reduced pressure, and the resulting crude product was refined through a column and recrystallized from toluene. It was filtered and then dried to obtain 19 g of a pale yellow powder.

FD-MS of the powder thus obtained was measured to obtain a peak of m/z = 1323 versus C₁₀₀H₆₆N₄ = 1322, and therefore this was identified as the intended compound (H18).

### Example 19 synthesis of compound (H19)

Mixed under argon flow were 10 g of the compound (A3), 21 g of the compound (B1), 6 g of sodium t-butoxide (manufactured by Hiroshima Wako Co., Ltd.), 1 g of bis(triphenylphosphine)palladium (II) chloride (manufactured by Tokyo Kasei Kogyo Co., Ltd.) and 500 ml of xylene, and they were reacted at 130°C for 24 hours.

After cooling, 1000 ml of water was added thereto, and the mixture was filtered through celite. The filtrate was extracted with toluene, and the extract was dried on anhydrous magnesium sulfate. This was concentrated under reduced pressure, and the resulting crude product was refined through a column and recrystallized from toluene. It was filtered and then dried to obtain 17 g of a pale yellow powder.

FD-MS of the powder thus obtained was measured to obtain a peak of m/z = 1123 versus C₈₄H₅₈N₄ = 1122, and therefore this was identified as the intended compound (H19).

### Example 20 synthesis of compound (H20)

Mixed under argon flow were 10 g of the compound (A3), 24 g of the compound (B4), 6 g of sodium t-butoxide (manufactured by Hiroshima Wako Co., Ltd.), 1 g of bis(triphenylphosphine)palladium (II) chloride (manufactured by Tokyo Kasei Kogyo Co., Ltd.) and 500 ml of xylene, and they were reacted at 130°C for 24 hours.

After cooling, 1000 ml of water was added thereto, and the mixture was filtered through celite. The filtrate was extracted with toluene, and the extract was dried on anhydrous magnesium sulfate. This was concentrated under reduced pressure, and the resulting crude product was refined through a column and recrystallized from toluene. It was filtered and then dried to obtain 22 g of a pale yellow powder.

FD-MS of the powder thus obtained was measured to obtain a peak of m/z = 1223 versus C₉₂H₆₂N₄ = 1222, and therefore this was identified as the intended compound (H20).

### Example 21 synthesis of compound (H21)

Mixed under argon flow were 10 g of the compound (A3), 21 g of the compound (B2), 6 g of sodium t-butoxide (manufactured by Hiroshima Wako Co., Ltd.), 1 g of bis(triphenylphosphine)palladium (II) chloride (manufactured by Tokyo Kasei Kogyo Co., Ltd.) and 500 ml of xylene, and they were reacted at 130°C for 24 hours.

After cooling, 1000 ml of water was added thereto, and the mixture was filtered through celite. The filtrate was extracted with toluene, and the extract was dried on anhydrous magnesium sulfate. This was concentrated under reduced pressure, and the resulting crude product was refined through a column and recrystallized from toluene. It was filtered and then dried to obtain 20 g of a pale yellow powder.

FD-MS of the powder thus obtained was measured to obtain a peak of m/z = 1123 versus C₈₄H₅₈N₄ = 1122, and therefore this was identified as the intended compound (H21).

### Example 22 synthesis of compound (H22)

Mixed under argon flow were 10 g of the compound (A3), 24 g of the compound (B7), 6 g of sodium t-butoxide (manufactured by Hiroshima Wako Co., Ltd.), 1 g of bis(triphenylphosphine)palladium (II) chloride (manufactured by Tokyo Kasei Kogyo Co., Ltd.) and 500 ml of xylene, and they were reacted at 130°C for 24 hours.

After cooling, 1000 ml of water was added thereto, and the mixture was filtered through celite. The filtrate was extracted with toluene, and the extract was dried on anhydrous magnesium sulfate. This was concentrated under reduced pressure, and the resulting crude product was refined through a column and recrystallized from toluene. It was filtered and then dried to obtain 18 g of a pale yellow powder.

FD-MS of the powder thus obtained was measured to obtain a peak of m/z = 1223 versus C₉₂H₆₂N₄ = 1222, and therefore this was identified as the intended compound (H22).

### Example 23 synthesis of compound (H23)

Mixed under argon flow were 10 g of the compound (A3), 24 g of the compound (B5), 6 g of sodium t-butoxide (manufactured by Hiroshima Wako Co., Ltd.), 1 g of bis(triphenylphosphine)palladium (II) chloride (manufactured by Tokyo Kasei Kogyo Co., Ltd.) and 500 ml of xylene, and they were reacted at 130°C for 24 hours.

After cooling, 1000 ml of water was added thereto, and the mixture was filtered through celite. The filtrate was extracted with toluene, and the extract was dried on anhydrous magnesium sulfate. This was concentrated under reduced pressure, and the resulting crude product was refined through a column and recrystallized from toluene. It was filtered and then dried to obtain 17 g of a pale yellow powder.

FD-MS of the powder thus obtained was measured to obtain a peak of m/z = 1223 versus C₉₂H₆₂N₄ = 1222, and therefore this was identified as the intended compound (H23).

### Example 24 synthesis of compound (H24)

Mixed under argon flow were 10 g of the compound (A3), 24 g of the compound (B3), 6 g of sodium t-butoxide (manufactured by Hiroshima Wako Co., Ltd.), 1 g of bis(triphenylphosphine)palladium (II) chloride (manufactured by Tokyo Kasei Kogyo Co., Ltd.) and 500 ml of xylene, and they were reacted at 130°C for 24 hours.

After cooling, 1000 ml of water was added thereto, and the mixture was filtered through celite. The filtrate was extracted with toluene, and the extract was dried on anhydrous magnesium sulfate. This was concentrated under reduced pressure, and the resulting crude product was refined through a column and recrystallized from toluene. It was filtered and then dried to obtain 16 g of a pale yellow powder.

FD-MS of the powder thus obtained was measured to obtain a peak of m/z = 1223 versus C₉₂H₆₂N₄ = 1222, and therefore this was identified as the intended compound (H24).

### Example 25 synthesis of compound (H25)

Mixed under argon flow were 10 g of the compound (A3), 27 g of the compound (B8), 6 g of sodium t-butoxide (manufactured by Hiroshima Wako Co., Ltd.), 1 g of bis(triphenylphosphine)palladium (II) chloride (manufactured by Tokyo Kasei Kogyo Co., Ltd.) and 500 ml of xylene, and they were reacted at 130°C for 24 hours.

After cooling, 1000 ml of water was added thereto, and the mixture was filtered through celite. The filtrate was extracted with toluene, and the extract was dried on anhydrous magnesium sulfate. This was concentrated under reduced pressure, and the resulting crude product was refined through a column and recrystallized from toluene. It was filtered and then dried to obtain 17 g of a pale yellow powder.

FD-MS of the powder thus obtained was measured to obtain a peak of m/z = 1323 versus C₁₀₀H₆₆N₄ = 1322, and therefore this was identified as the intended compound (H25).

### Example 26 synthesis of compound (H26)

Mixed under argon flow were 10 g of the compound (A3), 27 g of the compound (B9), 6 g of sodium t-butoxide (manufactured by Hiroshima Wako Co., Ltd.), 1 g of bis(triphenylphosphine)palladium (II) chloride (manufactured by Tokyo Kasei Kogyo Co., Ltd.) and 500 ml of xylene, and they were reacted at 130°C for 24 hours.

After cooling, 1000 ml of water was added thereto, and the mixture was filtered through celite. The filtrate was extracted with toluene, and the extract was dried on anhydrous magnesium sulfate. This was concentrated under reduced pressure, and the resulting crude product was refined through a column and recrystallized from toluene. It was filtered and then dried to obtain 21 g of a pale yellow powder.

FD-MS of the powder thus obtained was measured to obtain a peak of m/z = 1323 versus C₁₀₀H₆₆N₄ = 1322, and therefore this was identified as the intended compound (H26).

### Example 27 synthesis of compound (H27)

Mixed under argon flow were 10 g of the compound (A3), 30 g of the compound (B6), 6 g of sodium t-butoxide (manufactured by Hiroshima Wako Co., Ltd.), 1 g of bis(triphenylphosphine)palladium (II) chloride (manufactured by Tokyo Kasei Kogyo Co., Ltd.) and 500 ml of xylene, and they were reacted at 130°C for 24 hours.

After cooling, 1000 ml of water was added thereto, and the mixture was filtered through celite. The filtrate was extracted with toluene, and the extract was dried on anhydrous magnesium sulfate. This was concentrated under reduced pressure, and the resulting crude product was refined through a column and recrystallized from toluene. It was filtered and then dried to obtain 14 g of a pale yellow powder.

FD-MS of the powder thus obtained was measured to obtain a peak of m/z = 1423 versus C₁₀₈H₇₀N₄ = 1422, and therefore this was identified as the intended compound (H27).

### Example 28 synthesis of compound (H28)

Mixed under argon flow were 10 g of the compound (A3), 20 g of the compound (B10), 6 g of sodium t-butoxide (manufactured by Hiroshima Wako Co., Ltd.), 1 g of bis(triphenylphosphine)palladium (II) chloride (manufactured by Tokyo Kasei Kogyo Co., Ltd.) and 500 ml of xylene, and they were reacted at 130°C for 24 hours.

After cooling, 1000 ml of water was added thereto, and the mixture was filtered through celite. The filtrate was extracted with toluene, and the extract was dried on anhydrous magnesium sulfate. This was concentrated under reduced pressure, and the resulting crude product was refined through a column and recrystallized from toluene. It was filtered and then dried to obtain 19 g of a pale yellow powder.

FD-MS of the powder thus obtained was measured to obtain a peak of m/z = 1023 versus C₇₆H₅₄N₄ = 1022, and therefore this was identified as the intended compound (H28).

### Example 29 production of organic EL element

A glass substrate (manufactured by Geomatech Co., Ltd.) of 25 mm × 75 mm × 1.1 mm thickness equipped with an ITO transparent electrode was subjected to supersonic wave washing in isopropyl alcohol for 5 minutes and then to UV ozone washing for 30 minutes.

After washed, the glass substrate equipped with an ITO transparent electrode line was installed in a substrate holder of a vacuum deposition apparatus, and a film of the compound (H1) described above as a hole injecting material was formed in a film thickness of 60 nm on a face of a side at which the transparent electrode line was formed so that it covered the transparent electrode described above. This film (H1) functions as a hole injecting layer.

The following N,N,N',N'-tetra(4-biphenyl)-diaminobiphenylene film (hereinafter referred to as the TBDB film) having a film thickness of 20 nm was formed on the film (H1). This film functions as a hole transporting layer.

Further, EM1 was deposited on the TBDB film to form a film having a film thickness of 40 nm. Simultaneously with it, the following amine compound D1 having a styryl group was deposited as a light emitting molecule so that a weight ratio of EM1 to D1 was 40 : 2. This film functions as a light emitting layer.

The following Alq film having a film thickness of 10 nm was formed on the above film. This film functions as an electron injecting layer. Then, Li (Li source: manufactured by Saesgetter Co., Ltd.) which was a reducing dopant and Alq were subjected to binary deposition to form an Alq : Li film (film thickness: 10 nm) as an electron injecting layer (cathode). Metal Al was deposited on the above Alq : Li film to form a metal cathode, whereby an organic EL element was prepared.

The organic EL element thus obtained was measured for a voltage and a light emitting efficiency when an electric current of 1 mA/cm² was allowed to flow to determine a half life in light emission at an initial luminance of 1000 nit and a room temperature in DC constant electric current driving, and the results thereof are shown in Table 1. Further, observed was a light emitting state on the light emitting face when sending an electric current after storing at a storing temperature of 130°C for 100 hours, and the results thereof are shown in Table 1.

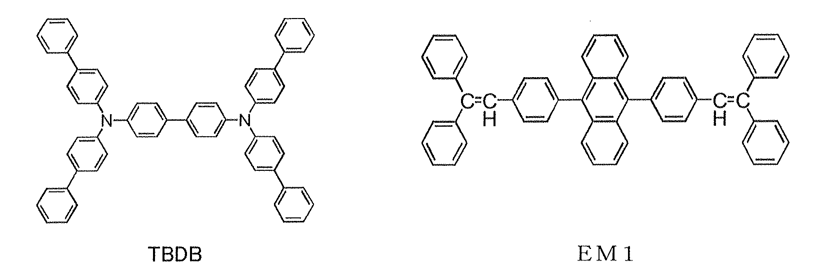

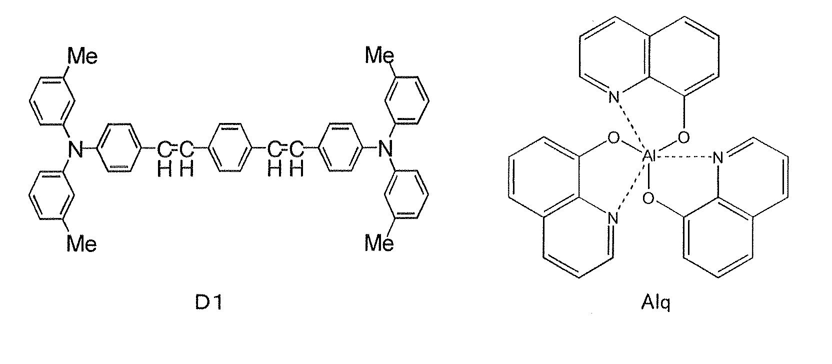

### Examples 30 to 56 production of organic EL elements

Organic EL elements were prepared in the same manner as in Example 29 to measure a light emitting efficiency and a half life and observe a light emitting state on the light emitting face after storing at a high temperature in the same manners, except that the compounds (H2) to (H28) described above were substituted respectively for the compound (H1). The results thereof are shown in Table 1.

### Comparative Examples 1 to 3 production of organic EL elements

Organic EL elements were prepared in the same manner as in Example 29 to measure a light emitting efficiency and a half life and observe a light emitting state on the light emitting face after storing at a high temperature in the same manner, except that the following compounds (H'1) to (H'3) were substituted respectively for the compound (H1). The results thereof are shown in Table 1.

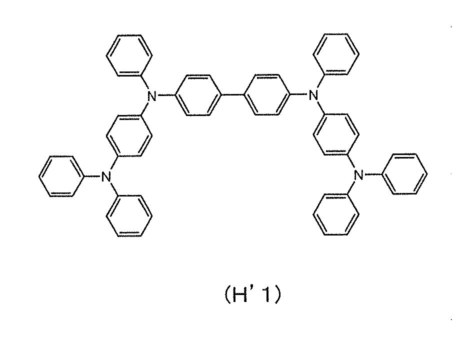

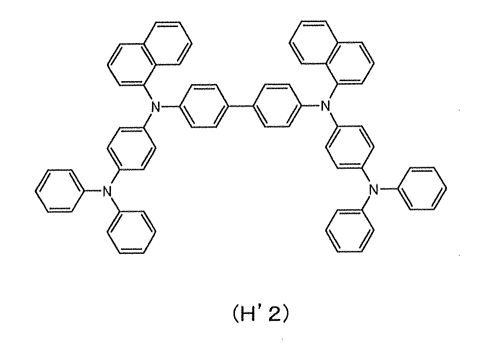

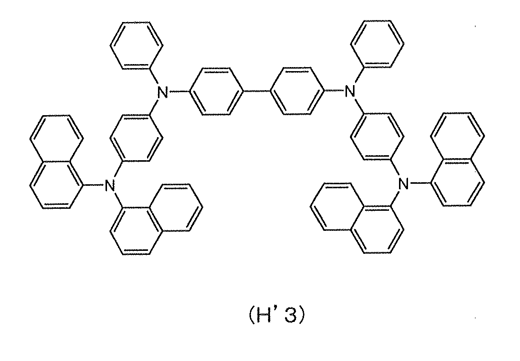

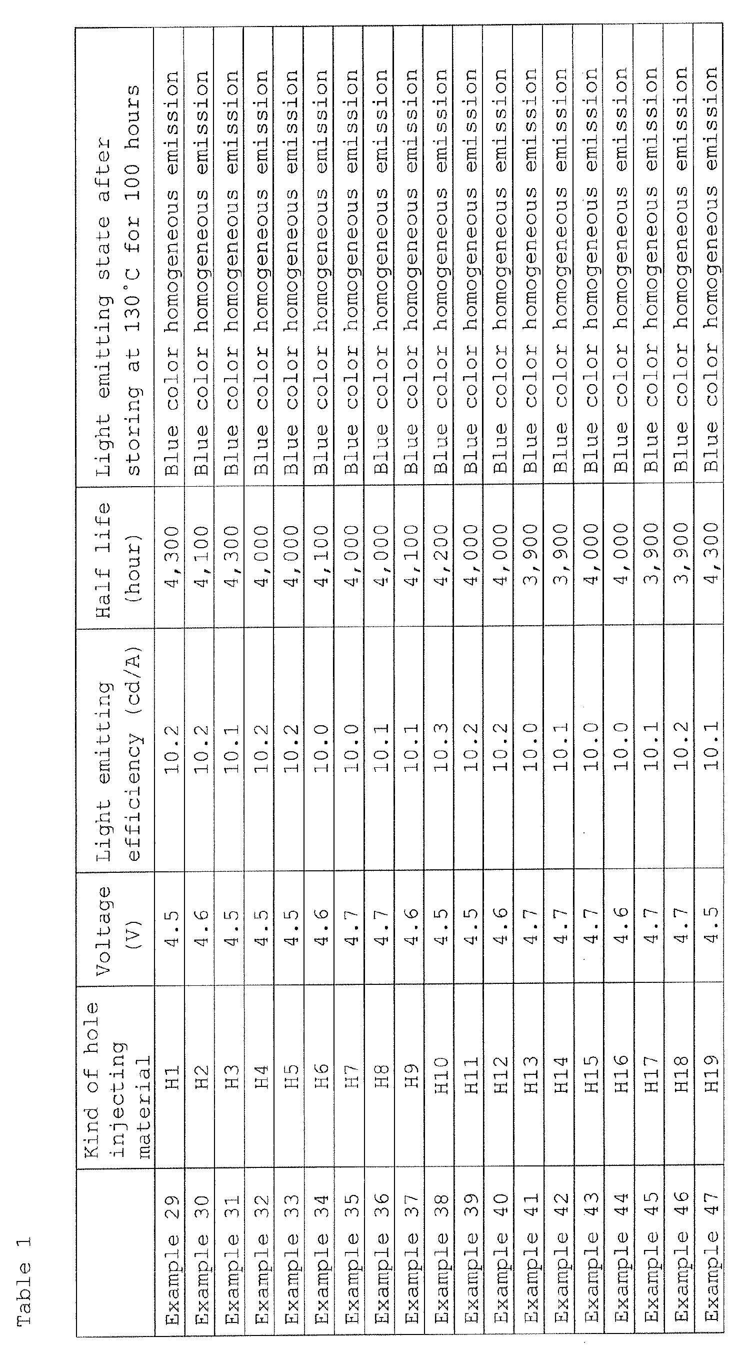

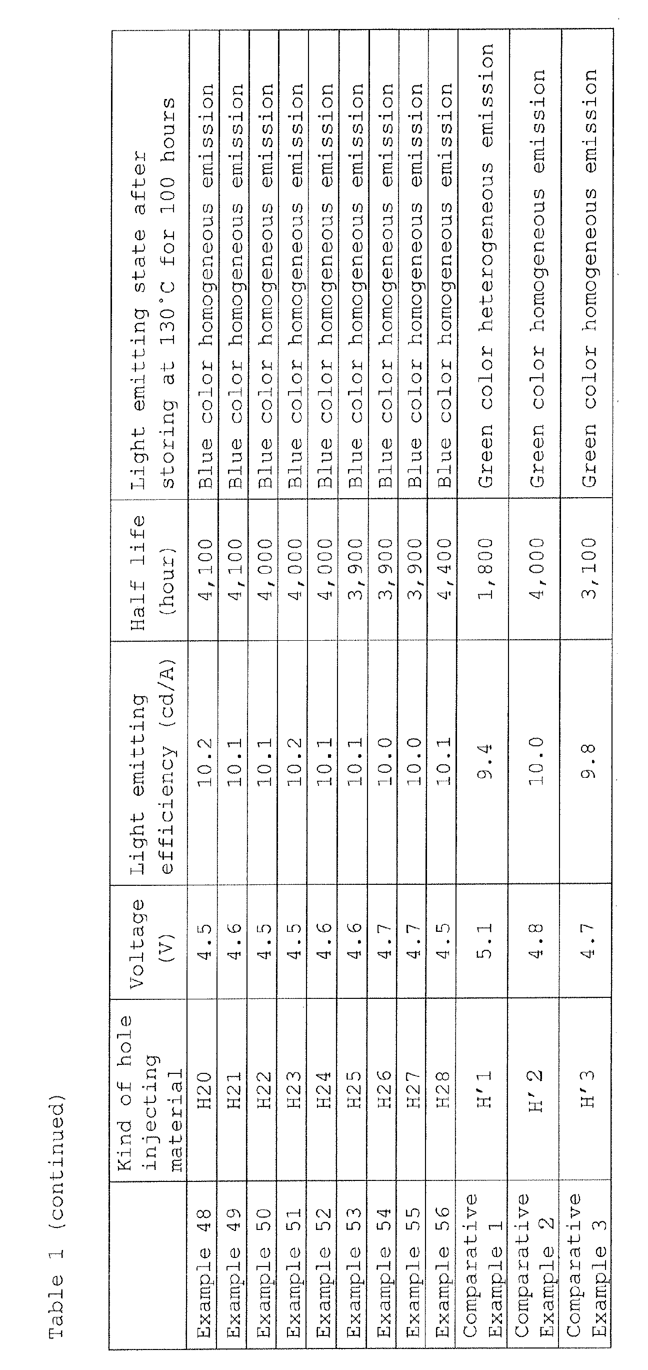

It can be found from the results shown in Table 1 that the elements using the aromatic amine derivatives of the present invention for the hole injecting material in Examples 29 to 56 were excellent in a storage stability at a high temperature while maintaining a high light emitting efficiency at a low voltage and a long life. In contrast with this, the organic EL elements prepared in Comparative Examples 1 to 3 were inferior in a heat resistance and therefore did not emit an essential blue color, and they heterogeneously emitted light or emitted light which was shifted to a longer wave length due to other light emitting components which were mixed in.

### INDUSTRIAL APPLICABILITY

As explained above in details, the aromatic amine derivatives of the present invention and the organic EL elements using the same make it possible to emit a blue color even under a high temperature while maintaining a high light emitting efficiency at a low voltage and a long life. Accordingly, they are very useful as an organic EL element used in environment in which a heat resistance and a high temperature storage stability are required, for example, an on-vehicle element.

## Claims

1. An aromatic amine derivative represented by the following Formula (1): 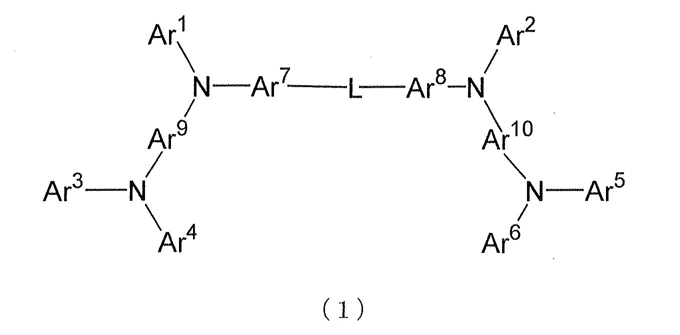 wherein Ar¹ to Ar² each represent a substituted or non-substituted condensed aryl group having 10 to 50 nuclear carbon atoms; Ar³ to Ar⁶ each represent a substituted or non-substituted aryl group having 6 to 50 nuclear carbon atoms; Ar⁷ to Ar¹⁰ each represent a substituted or non-substituted arylene group having 6 to 50 nuclear carbon atoms; substituents of Ar⁷ and Ar⁸ may form a ring;
L represents a single bond, an ether bond, a thioethers bond, a substituted or non-substituted arylene group having 6 to 50 nuclear carbon atoms, a substituted or non-substituted heteroarylene group having 6 to 50 nuclear carbon atoms, a substituted or non-substituted alkylene group having 1 to 50 carbon atoms or a substituted or non-substituted alkylidene group having 2 to 50 carbon atoms;
provided that the conditions of (1) and/or (2) are satisfied:
(1) at least one of Ar³ to Ar⁶ is a substituted or non-substituted condensed aryl group having 10 to 50 nuclear carbon atoms and
(2) at least one of Ar¹ to Ar² is a substituted or non-substituted condensed aryl group having 12 to 50 nuclear carbon atoms.

2. The aromatic amine compound as described in claim 1, wherein it is a hole injecting material.

3. An organic electroluminescent element in which an organic thin film layer comprising a single layer or plural layers including at least a light emitting layer is interposed between a cathode and an anode, wherein at least one layer of the above organic thin film layers contains the aromatic amine derivative as described in claim 1 in the form of a single component or a mixed component.

4. The organic electroluminescent element as described in claim 3, wherein the organic thin film layer described above has a hole transporting zone, and the above hole transporting zone contains the aromatic amine derivative as described in claim 1 in the form of a single component or a mixed component.

5. The organic electroluminescent element as described in claim 3, wherein the organic thin film layer described above has a hole injecting layer, and the above injecting layer contains the aromatic amine derivative as described in claim 1 in the form of a single component or a mixed component.

6. The organic electroluminescent element as described in any of claims 3 to 5, wherein it emits blue light.
